# EUROPEAN PATENT APPLICATION

(11) **EP 4 722 210 A1**
(43) Date of publication of application: **08.04.2026**
(21) Application number: 25823728.8
(22) Date of filing: 27.06.2025
(51) Int. Cl.: C07D 411/14, H01M 10/0567, H01M 10/0525

(54) **POLYCYCLIC COMPOUND AND PREPARATION METHOD THEREFOR, USE THEREOF, ELECTROLYTE, AND BATTERY**

(30) Priority: 08.07.2024 CN 202410906445
(71) Applicant: Guangzhou Tinci Materials Technology Co., Ltd., Guangzhou, Guangdong 510760 (CN)
(72) Inventor: FAN, Chaojun, Guangzhou, Guangdong 510670 (CN); FAN, Weizhen, Guangzhou, Guangdong 510670 (CN); SHI, Litao, Guangzhou, Guangdong 510670 (CN)
(74) Representative: Studio Torta S.p.A.
(86) International application number: PCT/CN2025/104817
(87) International publication number: WO 2026/012173

(57) **Abstract**

Provided are a polycyclic compound and a method for preparing the same, use, an electrolyte, and a battery. The polycyclic compound includes a compound represented by Formula I. In the compound represented by Formula I, a content of a cis-isomer represented by Formula II is greater than or equal to 98 wt%, 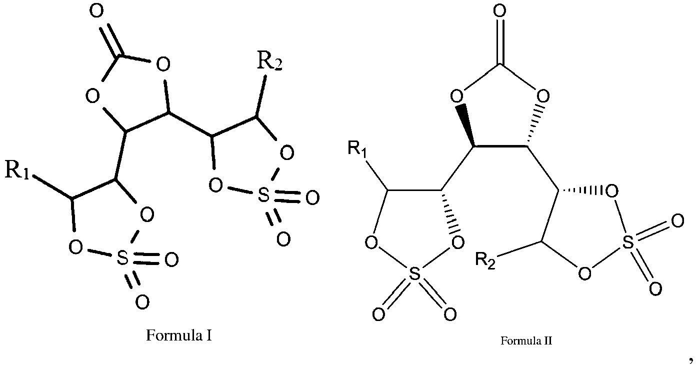 R₁ and R₂ are each independently selected from any one of H, F, alkyl, or fluoroalkyl.

## Description

### CROSS-REFERENCE TO RELATED APPLICATION

This application claims priority to Chinese patent application No. 202410906445X, titled "POLYCYCLIC COMPOUND AND METHOD FOR PREPARING THE SAME, USE, ELECTROLYTE, AND BATTERY", filed with China National Intellectual Property Administration on July 8, 2024, the entire contents of which are incorporated herein by reference.

### FIELD

The present disclosure belongs to the field of a battery, and more particularly, to a polycyclic compound and a method for preparing the same, use, an electrolyte, and a battery.

### BACKGROUND

With the rapid development of markets such as an electronic device, an electric vehicle, smart home, a power tool, and intelligent transportation, a demand for a battery is continuously increasing. Taking a lithium-ion battery as an example, the lithium-ion battery is widely used in consumer electronics, energy storage and power batteries, the smart home, and other fields due to advantages of high specific energy, long cycle life, and low self-discharge thereof. Generally, the battery consists of a positive electrode sheet, a negative electrode sheet, a separator, and an electrolyte. The electrolyte usually includes a solvent, an electrolyte lithium salt, and an electrolyte additive. Performance of the battery can be improved by adding the electrolyte additive to the electrolyte. Selecting an appropriate additive has an important impact on exertion of electrochemical performance of the battery.

### SUMMARY

The present disclosure aims to solve one of the technical problems in the related art to some extent.

The technical solution of the present disclosure is completed by the inventor based on the following findings.

Currently, with increasing requirements for battery endurance and a further increase in a cost pressure of a battery cell, selection of a transition metal in a positive electrode active material becomes a mainstream development direction. For example, NCM (lithium nickel cobalt manganese oxide), LNMO (lithium nickel manganese oxide), or LMFP (lithium manganese iron phosphate) begin to develop continuously in the fields of power and energy storage. However, these materials all have a common problem: with the progress of cycling, due to intrinsic stability of the material itself or an influence of an acidic component in the electrolyte, transition metal dissolution may occur. On the one hand, the dissolved transition metal may lead to structural collapse and capacity fading of a positive electrode material. On the other hand, the dissolved transition metal may also catalyze an electrolyte reaction, resulting in high-temperature gas generation and poor interface stability. As an important component of a lithium secondary battery, the electrolyte has an important impact on comprehensive performance of the battery. An additive is an important component of the electrolyte. A suitable additive can significantly improve electrochemical performance of the battery. Therefore, it is of great importance to select the suitable additive for reducing a negative impact of the transition metal on the electrochemical performance of the battery. It was found by the inventor that applying a compound represented by Formula I as the additive to a lithium-ion battery electrolyte has a good effect on improving the electrochemical performance of the battery. Specifically, not only formation of a dense CEI film on the positive electrode and a dense SEI film on the negative electrode can be facilitated, but also a small amount of inorganic salts (taking the lithium-ion battery as an example, the inorganic salts include Li₂SO₄ and/or Li₂O) may be formed in the CEI film formed by decomposition of the positive electrode. In this way, rigidity and flexibility of a positive electrode interface layer can be improved, inhibiting electrolyte decomposition, and enhancing stability of a positive electrode interface film. In addition, the CEI film formed by the compound represented by Formula I can also reduce electrochemical impedance of the positive electrode, accelerate a transport rate of active ions (such as Li⁺). Also, forming a more stable electrode-electrolyte interface can be facilitated, reducing internal resistance and an interface reaction of the battery, and achieving protection for the positive electrode. Further, dissolution of transition metal ions from an active material of a positive electrode sheet can also be inhibited, reducing cyclic capacity fading of the battery, and improving cycling stability. Moreover, lithium carbonate may be generated in the SEI film formed by the compound represented by Formula I on the negative electrode. The lithium carbonate helps to improve film-forming uniformity and stability of the SEI film. In addition, the compound represented by Formula I has low impedance, which is also conducive to reducing battery impedance.

However, based on a conventional synthesis process and selection of a raw material for the compound represented by Formula I, the target product usually contains different conformations and other impurity components. The different conformations include a cis-isomer represented by Formula II and a trans-isomer represented by Formula III. It was found by the inventor that the cis-isomer represented by Formula II is easy to gain electrons and be reduced, leading to cleavage of an S-O single bond. In a transition state formed after the cleavage, three O atoms from O, S=O, and S=O on another cyclic sulfate group may form a claw-like spatial structure, which may easily capture metal ions and form a cage-like complex. Therefore, the already dissolved transition metal can be stabilized, reducing catalytic decomposition side reaction of the transition metal on the SEI film, and inhibiting a negative impact caused by deposition of the transition metal in an elemental form after being reduced at the negative electrode. However, even if the trans-isomer represented by Formula III undergoes bond rotation, a spatial distance between substituted or unsubstituted DTD (i.e., substituted or unsubstituted ethylene sulfate) rings on two sides in the trans-isomer represented by Formula III is still larger than that of the cis-isomer. Overlap between the substituted or unsubstituted DTD rings on two sides is weakened, making it difficult to form a suitable intermediate-state cage-like structure to capture and stabilize the transition metal. That is, the trans-isomer represented by Formula III cannot effectively eliminate a side reaction caused by the transition metal, which may reduce an improvement effect of the compound represented by Formula I on battery performance.

In view of this, a first objective of the present disclosure is to provide a polycyclic sulfate product. A target product of this product is mainly in a cis-isomer, which can reduce an impact of transition metal dissolution on the battery performance on the basis of improving the battery performance.

In a first aspect, the present disclosure provides a polycyclic compound. The polycyclic compound includes a compound represented by Formula I. In the compound represented by Formula I, a content of a cis-isomer represented by Formula II is greater than or equal to 98 wt%, 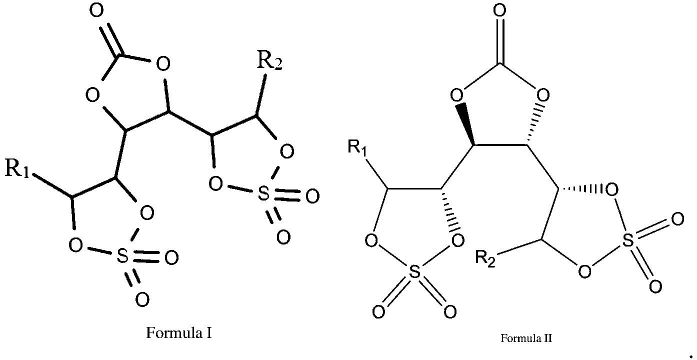 R₁ and R₂ are each independently selected from any one of H, F, alkyl, or fluoroalkyl.

The above polycyclic compound of the present disclosure can provide the following advantageous effects. By controlling a content of the cis-isomer represented by Formula II in the compound represented by Formula I to be greater than or equal to 98 wt% in the present disclosure, a problem that the improvement effect on the battery performance decreases due to presence of the trans-isomer in the compound represented by Formula I can be effectively alleviated. Further, when the polycyclic compound is used as an electrolyte additive in the battery, the electrochemical performance of the battery can be well improved, and enhanced high-temperature performance and cycling performance can be achieved.

In some embodiments, the polycyclic compound satisfies at least one of the following conditions: (i) the polycyclic compound further includes a chlorine-containing organic compound, based on a mass of the polycyclic compound, a total content of the chlorine-containing organic compound being less than or equal to 300 ppm; (ii) the polycyclic compound further includes other organic compounds being selected from one or more of the following 6 compounds: 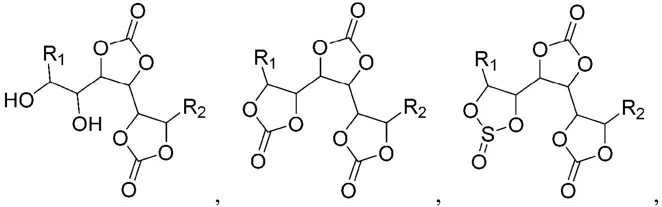 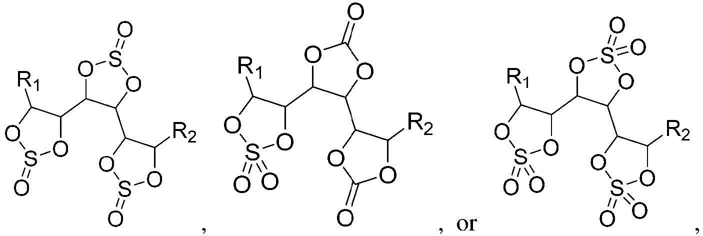 based on the mass of the polycyclic compound, a total content of the other organic compounds being less than or equal to 1000 ppm; (iii) based on the mass of the polycyclic compound, a content of the compound represented by Formula I is greater than or equal to 99 wt%. The polycyclic compound has high purity, which can further enhance the improvement effect on the electrochemical performance of the battery; (iv) in the compound represented by Formula I, the content of the cis-isomer represented by Formula II is greater than or equal to 98.9 wt%. Therefore, the improvement effect of the polycyclic compound on the electrochemical performance of the battery can be further enhanced; (v) the compound represented by Formula I further includes a trans-isomer represented by Formula III, in the compound represented by Formula I, a content of the trans-isomer represented by Formula III being less than or equal to 1%, 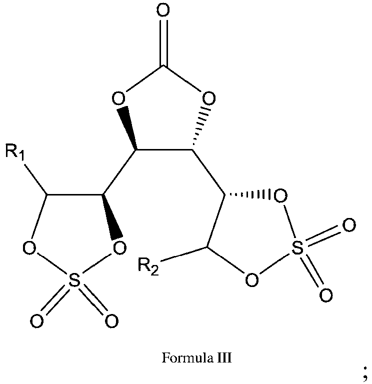 or (vi) R₁ and R₂ are each independently selected from any one of H, F, or fluoroalkyl.

In some embodiments, the polycyclic compound satisfies at least one of the following conditions: (a) the chlorine-containing organic compound includes a chlorine-containing organic compound without cyclic sulfate groups and cyclic carbonate groups, a chlorine-containing organic compound containing only cyclic carbonate groups, a chlorine-containing organic compound containing only cyclic sulfate groups, or a chlorine-containing organic compound containing 1 to 2 cyclic carbonate groups and 1 cyclic sulfate group; (b) based on the mass of the polycyclic compound, the total content of the chlorine-containing organic compound is less than or equal to 100 ppm. Therefore, a problem of a reduced improvement effect of the compound represented by Formula I on the electrochemical performance of the battery, caused by presence of chlorine-containing organic impurities, can be further improved; (c) based on the mass of the polycyclic compound, the total content of the other organic compounds is less than or equal to 200 ppm. Therefore, the problem of the reduced improvement effect of the compound represented by Formula I on the electrochemical performance of the battery, caused by presence of the impurities, can be further improved; (d) based on the mass of the polycyclic compound, the content of the compound represented by Formula I is greater than or equal to 99.9 wt%. Therefore, the improvement effect of the polycyclic compound on the electrochemical performance of the battery can be further enhanced; or (e) in the compound represented by Formula I, the content of the trans-isomer represented by Formula III is less than or equal to 0.5 wt%. Therefore, a problem that an improvement effect of the compound represented by Formula I on the electrochemical performance of the battery decreases due to presence of the trans-isomer can be further alleviated.

In some embodiments, the content of the cis-isomer represented by Formula II in the compound represented by Formula I is less than or equal to 99.99 wt%; and/or the content of the trans-isomer represented by Formula III in the compound represented by Formula I is greater than or equal to 0.01 wt%. Therefore, on the premise of ensuring that the problem that the improvement effect of the compound represented by Formula I on the electrochemical performance of the battery decreases due to presence of the trans-isomer is alleviated, a cost can be reduced.

In some embodiments, the polycyclic compound satisfies at least one of the following conditions: (α) the chlorine-containing organic compound includes one or more of 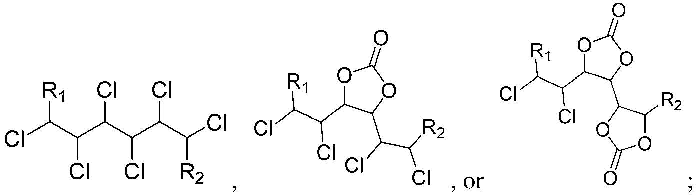 (β) based on the mass of the polycyclic compound, the total content of the chlorine-containing organic compound is less than or equal to 50 ppm. Therefore, the problem of a reduced improvement effect of the compound represented by Formula I on the electrochemical performance of the battery, caused by presence of chlorine-containing organic impurities, can be further improved; (γ) in the compound represented by Formula I, the content of the cis-isomer represented by Formula II is greater than or equal to 99.9 wt%. Therefore, the improvement effect of the polycyclic compound on the electrochemical performance of the battery can be further enhanced; (δ) in the compound represented by Formula I, the content of the trans-isomer represented by Formula III is less than or equal to 0.1%. Therefore, the improvement effect of the polycyclic compound on the electrochemical performance of the battery can be further enhanced; (ε) the compound represented by Formula I includes 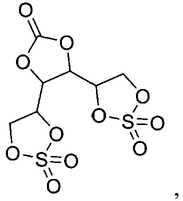 and the compound represented by Formula II includes 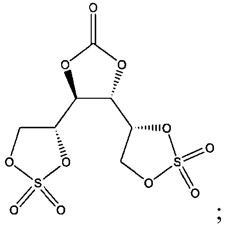 (ζ) in the compound represented by Formula I, the content of the trans-isomer represented by Formula III is greater than or equal to 0.01 wt%; or (η) in the compound represented by Formula I, the content of the cis-isomer represented by Formula II is less than or equal to 99.99 wt%.

Based on the same inventive concept as the first aspect of the present disclosure, it is desired to obtain a polycyclic compound product in which purity of the cis-isomer represented by Formula II in the compound represented by Formula I is greater than or equal to 98 wt%. For this purpose, a method for preparing the polycyclic compound according to a second aspect of the present disclosure is developed.

In the preparation of the compound represented by Formula I using an existing synthesis process, there are common problems such as a high content of a by-product relative to the target product, a low synthesis efficiency, and a low yield. For example, there is currently a method in which a hexahydric alcohol and a carbonate undergo transesterification under catalysis of a catalyst, and then sulfonyl chloride is added for recrystallization to obtain the target product. However, this method has poor selectivity, making it difficult to obtain a single target product, with a low yield of the target product and generation of many impurities. Further, being affected by purity and conformation of a hexahydric alcohol raw material, a content of by-product in the target product may also be affected, affecting the improvement effect on the electrochemical performance of the battery. In the present disclosure, by selecting a specific hexahydric alcohol raw material and controlling a content of other configurations in the hexahydric alcohol raw material, a purpose of the second aspect may be achieved.

In a second aspect, the present disclosure provides a method for preparing a polycyclic compound. The method includes: (1) mixing a hexahydric alcohol, a monohydric alcohol, a carbonate, and a basic catalyst, and performing a transesterification reaction, to obtain a compound 1; (2) dissolving the compound 1 in a solvent, and performing a condensation reaction with thionyl chloride, to obtain a compound 2; and (3) reacting the compound 2 with an oxidizing agent, to obtain the compound represented by Formula I. Structural formulas of the compound 1 and the compound 2 are represented by 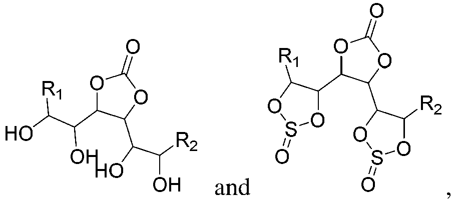 respectively. The hexahydric alcohol includes 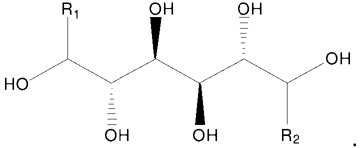 A mass percentage of 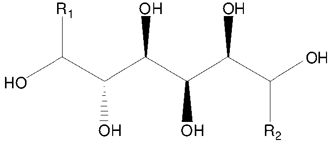 in the hexahydric alcohol is less than or equal to 2%. R₁ and R₂ are each independently selected from any one of H, F, alkyl, or fluoroalkyl.

In the present disclosure, the method for preparing the polycyclic compound can provide the following advantageous effects. The hexahydric alcohol has high flexibility and a strong intermolecular force. Also, 4 carbon atoms in the hexahydric alcohol are chiral, resulting in multiple possibilities for molecular conformation of the hexahydric alcohol. Taking the case where both R₁ and R₂ are H as an example, i.e., using the hexahydric alcohol 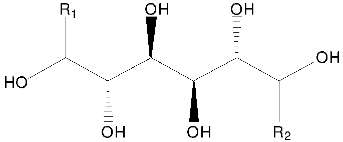 (mannitol) as the raw material in the present disclosure, a current industrial method for producing this type of hexahydric alcohol mainly includes a kelp extraction method and a catalytic hydrogenation method. The catalytic hydrogenation method is a main international production method for mannitol. A main reason is that the kelp purification method involves cumbersome processes for refining and removing impurities such as polysaccharides, with a low yield, a high manufacturing cost, and a raw material source restricted by regions and seasons. Considering a raw material cost, the catalytic hydrogenation method usually uses low-cost sucrose as the raw material, which undergoes hydrolysis, isomerization, and other processes to obtain sucrose, fructose, or glucose-fructose syrup. This product is then used as the raw material for catalytic hydrogenation. However, regardless of the raw material used to produce mannitol, presence of sorbitol is unavoidable. A mannitol product prepared by a current mainstream method contains sorbitol in an amount substantially equivalent to that of mannitol. Also, since mannitol and sorbitol may form a eutectic, separation thereof may be difficult. Purification processes of concentration, cooling crystallization, separation, and drying may increase a content of mannitol. In addition, the more purification cycles are performed, the higher purity of mannitol obtained, and the higher corresponding cost of a mannitol raw material. Therefore, from a cost perspective, except for special fields such as medicine and food where mannitol has proven effects while sorbitol does not and thus a content of sorbitol in mannitol is regulated, other fields especially the chemical industry do not impose special requirements on the content of sorbitol in mannitol to reduce the manufacturing cost. However, since those skilled in the art do not recognize an impact of the trans-isomer represented by Formula III in the compound represented by Formula I on performance, a content of 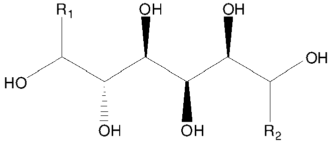 in the hexahydric alcohol may not be controlled. In the present disclosure, by using the hexahydric alcohol 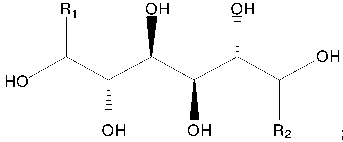 as the raw material and controlling a mass percentage of 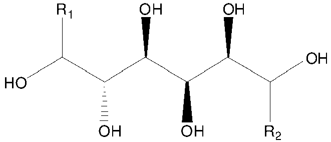 in the hexahydric alcohol to be less than or equal to 2%, the finally prepared compound represented by Formula I may be dominated by the cis-isomer represented by Formula II, which can greatly reduce a content of the trans-isomer represented by Formula III in the final product. In other words, generation of a trans-isomer by-product may be inhibited from the perspective of raw material conformation. Therefore, a problem of a reduced improvement effect of the compound represented by Formula I on the electrochemical performance of the battery, caused by presence of the trans-isomer in the compound represented by Formula I, can be effectively alleviated. Further, the improvement effect of the prepared polycyclic compound on the electrochemical performance of the battery (such as high-temperature performance and cycling performance) can be enhanced.

In some embodiments, said mixing the hexahydric alcohol, the monohydric alcohol, the carbonate, and the basic catalyst, and performing the transesterification reaction satisfies at least one of the following conditions: (A) subsequent to completion of the transesterification reaction, the method further includes: performing a reduced-pressure treatment to remove a low-boiling component; (B) the transesterification reaction is performed at a temperature ranging from 65°C to 100°C under a pressure controlled to be less than or equal to 2.5 MP for 0.5 hours to 6 hours. Further, the transesterification reaction is performed at a temperature ranging from 70°C to 90°C under a pressure ranging from 0.5 MP to 2.2 MP for 0.5 hours to 6 hours. Still further, the transesterification reaction is performed at a temperature ranging from 75°C to 85°C under a pressure ranging from 1 MP to 2 MP. Therefore, both a relatively high raw material conversion rate and a suitable transesterification reaction rate can be balanced, obtaining a compound 1 with relatively high selectivity and yield, and a low impurity content; (C) a molar ratio of the carbonate to the hexahydric alcohol ranges from 3:1 to 5:1. Therefore, not only the suitable transesterification reaction rate and the high conversion rate can be achieved, but also the impurity content in the final product can be reduced; (D) a molar ratio of the monohydric alcohol to the hexahydric alcohol ranges from 10:1 to 20:1. Further, the molar ratio of the monohydric alcohol to the hexahydric alcohol ranges from 12:1 to 18:1. Still further, the molar ratio of the monohydric alcohol to the hexahydric alcohol ranges from 14:1 to 16:1. In the present disclosure, the monohydric alcohol serves as the solvent on the one hand, and an addition amount of the monohydric alcohol usually only needs to meet a requirement of completely dissolving the reaction raw material of the hexahydric alcohol and the carbonate. Generally, when the molar ratio of the monohydric alcohol to the hexahydric alcohol is greater than or equal to 10, the dissolution requirement may be met. The related art mentions that excessive addition of solvent methanol is acceptable for dissolving the reaction raw material. However, actually it was found by the inventor that a content of monohydric alcohol also affects selectivity of the transesterification reaction. If the content of monohydric alcohol is too high (for example, when the molar ratio of the monohydric alcohol to the hexahydric alcohol is greater than 20:1), the selectivity of the transesterification reaction may be reduced to a certain extent. Based on this, controlling the molar ratio of the monohydric alcohol to the hexahydric alcohol to be (10:1) to (20:1), or (12:1) to (18:1), or (14:1) to (16:1) is not only beneficial for achieving the suitable transesterification reaction rate and the high conversion rate, but also beneficial for reducing the impurity content in the final product; (E) based on a mass of the hexahydric alcohol, an amount of the basic catalyst ranges from 0.01 wt% to 5 wt%. Optionally, the amount of the basic catalyst ranges from 0.02 wt% to 0.5 wt%, and further optionally from 0.02 wt% to 0.1 wt%. Therefore, not only smooth progress of the transesterification reaction can be facilitated, but also a risk of an increased amount of thionyl chloride and excessive generation of sulfur dioxide in the subsequent condensation reaction caused by an excessive amount of the basic catalyst can be reduced; (F) the carbonate includes one or more of dimethyl carbonate, diethyl carbonate, ethyl methyl carbonate, or diphenyl carbonate; (G) the monohydric alcohol includes one or more of methanol, ethanol, propanol, isopropanol, butanol, or tert-butanol; (H) the basic catalyst includes one or more of sodium hydroxide, potassium hydroxide, potassium carbonate, sodium carbonate, lithium carbonate, sodium methoxide, sodium ethoxide, sodium tert-butoxide, potassium tert-butoxide, triethylamine, or pyridine; or (I) a mass percentage of 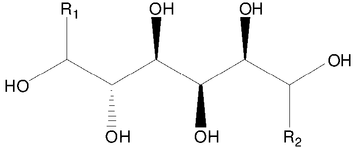 in the hexahydric alcohol is less than or equal to 1%. Further, the mass percentage of 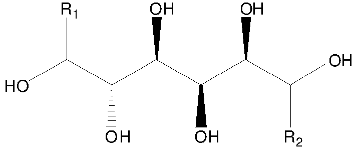 in the hexahydric alcohol is less than or equal to 0.5%. Further, the mass percentage of 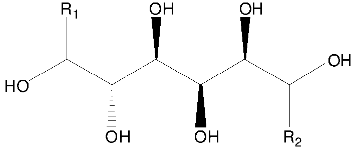 in the hexahydric alcohol is less than or equal to 0.1%;

In some embodiments, the mass percentage of 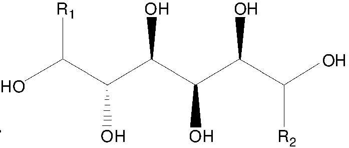 in the hexahydric alcohol is greater than or equal to 0.01%.

In some embodiments, the method satisfies at least one of the following conditions: (I) in the step (2), subsequent to completion of the condensation reaction, the method further includes: performing a reflux deacidification treatment. Therefore, the residual HCl generated from the condensation reaction can be reduced. Optionally, a temperature of the reflux deacidification treatment ranges from 40°C to 80°C; and/or a duration of the reflux deacidification treatment ranges from 10 minutes to 50 minutes. Meeting the above reflux deacidification treatment conditions is beneficial for further reducing the residual HCl generated from the esterification reaction; (II) in the step (2), subsequent to dissolving the compound 1 in the solvent, the condensation reaction is performed by adding thionyl chloride dropwise. Therefore, formation of the impurity 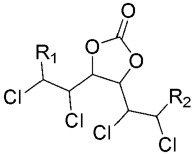 can be well inhibited; (III) in the step (2), the condensation reaction is performed at a temperature ranging from 20°C to 100°C for 1 hour to 4 hours. Therefore, a reaction efficiency and the purity of the final product can be well balanced; (IV) in the step (2), a molar ratio of the compound 1 to the thionyl chloride is 1:(2 to 3), and an addition duration for the thionyl chloride ranges from 0.5 hours to 2 hours. Therefore, not only the reaction rate can be balanced, but also purity of the compound 2 and the final product can be improved, reducing the impurity content; (V) in the step (2), the solvent includes one or more of an ether solvent, N,N-dimethylformamide, N,N-dimethylacetamide, tetrahydrofuran, or an ester solvent; (VI) in the step (3), the oxidizing agent is provided as an aqueous solution, and subsequent to reacting the compound 2 with the oxidizing agent, the method further includes: performing a heating treatment on a reaction product to remove water; or (VII) subsequent to completion of the transesterification reaction, the method further includes: performing the reduced-pressure treatment to remove the low-boiling component. Therefore, the purity of the compound represented by Formula I can be further improved, and the impurity content can be further reduced.

In a third aspect, the present disclosure provides a polycyclic compound prepared by the above method preparing the polycyclic compound. The prepared polycyclic compound includes a compound represented by Formula I. In the compound represented by Formula I, a content of a cis-isomer represented by Formula II is greater than or equal to 98 wt%. Characteristics and effects described for the above method for preparing the polycyclic compound are equally applicable to this polycyclic compound, and will not be repeated herein.

In a fourth aspect, the present disclosure provides use of the polycyclic compound according to the above or the above method for preparing the polycyclic compound in the field of electrolyte and battery.

In a fifth aspect, the present disclosure provides an electrolyte. The electrolyte includes an electrolyte additive. The electrolyte additive includes the polycyclic compound according to the above or the polycyclic compound prepared by the method according to the above. Using this electrolyte in the battery can improve the high-temperature performance and cycling performance of the battery well.

In some embodiments, based on a total mass of the electrolyte, a content of the electrolyte additive ranges from 0.1 wt% to 5 wt%.

In a sixth aspect, the present disclosure provides a battery. The battery includes the electrolyte according to the above.

Additional aspects and advantages of the present disclosure will be provided at least in part in the following description, or will become apparent at least in part from the following description, or can be learned from practicing of the present disclosure.

### BRIEF DESCRIPTION OF THE DRAWINGS

The above and/or additional aspects and advantages of the present disclosure will become more apparent and more understandable from the following description of embodiments taken in conjunction with the accompanying drawings.
FIG. 1 is a flow chart of preparation of a polycyclic compound according to an embodiment of the present disclosure.
FIG. 2 is a hydrogen nuclear magnetic resonance spectrum of a final product prepared according to Example 1 of the present disclosure.
FIG. 3 is a hydrogen nuclear magnetic resonance spectrum of a final product prepared according to Comparative Example 3 of the present disclosure.
FIG. 4 is an HPLC chromatogram of crude mannitol, in which 1 indicates a peak position at 2.723 min corresponding to mannitol, while 2 indicates a peak position at 3.944 min corresponding to sorbitol.
FIG. 5 is an HPLC chromatogram of a polycyclic compound obtained in Example 1, in which 3 indicates a peak position at 9.883 min corresponding to a cis-polycyclic compound, while 4 indicates a peak position at 10.523 min corresponding to a trans-polycyclic compound.

### DETAILED DESCRIPTION

Embodiments of the present disclosure are described below in detail, which are intended to explain, rather than limiting, embodiments of the present disclosure.

In a first aspect, the present disclosure provides a polycyclic compound. The polycyclic compound includes a compound represented by Formula I. In the compound represented by Formula I, a content of a cis-isomer represented by Formula II is greater than or equal to 98 wt%, 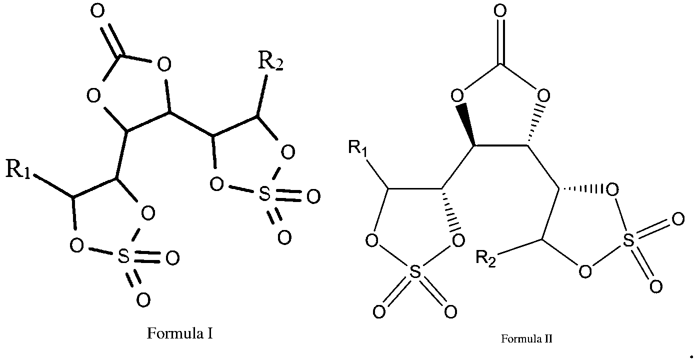 R₁ and R₂ are each independently selected from any one of H, F, alkyl, or fluoroalkyl.

It should be noted that R₁ and R₂ may be the same or different. The alkyl and fluoroalkyl may each independently be an alkyl group or a fluoroalkyl group with 1 to 10 carbon atoms. In addition, the fluoroalkyl may be an alkyl group partially substituted with fluorine or fully substituted with fluorine. Taking the case where both R₁ and R₂ are H as an example, in this polycyclic compound, the compound represented by Formula I is dominated by the cis-polycyclic compound structure represented by Formula II (that is, with a plane where the carbon skeleton is located as a reference, DTD rings being at a same side of the plane). The content of the cis-isomer represented by Formula II is greater than or equal to 98 wt%. Exemplarily, the content of the cis-isomer represented by Formula II in the compound represented by Formula I may be 98 wt%, 98.2 wt%, 98.4 wt%, 98.5 wt%, 98.7 wt%, 98.9 wt%, 99 wt%, 99.2 wt%, 99.4 wt%, 99.5 wt%, 99.7 wt%, 99.9 wt%, 99.92 wt%, 99.95 wt%, 99.97 wt%, or 99.99 wt%.

The compound represented by Formula I may be used as an additive in an electrolyte to improve electrochemical performance of a battery. Specifically, in a battery formation stage, the compound represented by Formula I tends to form a dense CEI film on a positive electrode and a dense SEI film on a negative electrode. The CEI film formed by decomposition of the compound on the positive electrode contains a small amount of inorganic salts (Taking a lithium battery as an example, these inorganic salts include Li₂SO₄ and/or Li₂O). As a result, rigidity and flexibility of a positive electrode interface layer can be improved, electrolyte decomposition can be inhibited, and stability of a positive electrode interface film can be improved. In addition, the CEI film formed by the compound represented by Formula I can also reduce electrochemical impedance of the positive electrode, accelerate a transport rate of active ions (e.g., Li⁺), and help form a more stable electrode-electrolyte interface. In this way, battery internal resistance and an interfacial reaction can be reduced, providing protection for the positive electrode. In addition, dissolution of transition metal ions from an active material of a positive electrode sheet can be inhibited, reducing cyclic capacity fading of the battery, and improving cycling stability. Moreover, lithium carbonate is generated in the SEI film formed by the compound represented by Formula I on the negative electrode. The lithium carbonate helps improve film-forming uniformity and stability of the SEI film. In addition, the compound represented by Formula I easily accepts electrons and is reduced, leading to cleavage of an S-O single bond. In a transition state formed after the cleavage, three oxygen atoms from O, S=O, and S=O on another cyclic sulfate group may form a claw-like spatial structure. This structure readily captures metal ions and forms a cage-like complex, which can stabilize the already dissolved transition metal, reduce a catalytic decomposition side reaction of the transition metal on the SEI film, and inhibit a negative effect caused by deposition of the transition metal in an elemental form after being reduced on the negative electrode. Furthermore, the compound represented by Formula I has low impedance, which is also beneficial for reducing battery impedance.

Based on a conventional synthesis process and selection of the raw material for the compound represented by Formula I, the target product usually contains different conformations and other impurity components. The different conformations include the cis-isomer represented by Formula II and the trans-isomer represented by Formula III. It was found by the inventor that the cis-isomer represented by Formula II tends to gain electrons and be reduced, leading to the cleavage of the S-O single bond. In the transition state formed after the cleavage, the three O atoms from O, S=O, and S=O on another cyclic sulfate group can form the claw-like spatial structure, which easily captures metal ions to form the cage-like complex. Therefore, the already dissolved transition metal can be stabilized, the catalytic decomposition side reaction of the transition metal on the SEI film can be reduced, and the negative effect caused by deposition of the transition metal in the elemental form after being reduced on the negative electrode can be inhibited. However, even if the trans-isomer represented by Formula III undergoes bond rotation, a spatial distance between substituted or unsubstituted DTD (i.e., substituted or unsubstituted ethylene sulfate) rings on two sides of the trans-isomer is still larger than that of the cis-isomer. Overlap of the substituted or unsubstituted DTD rings on two sides is weakened, making it difficult to form a suitable intermediate cage-like structure to capture and stabilize the transition metal. That is, the trans-isomer represented by Formula III cannot effectively eliminate a side reaction caused by the transition metal, which may reduce an improvement effect of the compound represented by Formula I on battery performance.

In the present disclosure, by controlling the content of the cis-isomer represented by Formula II in the compound represented by Formula I to be greater than or equal to 98 wt%, a problem of a reduced improvement effect on the battery performance caused by the presence of the trans-isomer in the compound represented by Formula I can be effectively alleviated. Further, when the polycyclic compound is used as an electrolyte additive in the battery, electrochemical performance of the battery can be better improved, achieving enhanced high-temperature performance and cycling performance.

In some specific embodiments of the present disclosure, when at least one of R₁ or R₂ is not H, synthesis of the compound represented by Formula I may be performed by pre-obtaining an alcohol raw material with a corresponding substitution position and a substituent type, then using these alcohol raw materials to synthesize the compound represented by Formula I through processes such as a transesterification reaction and a condensation reaction.

In some specific embodiments of the present disclosure, R₁ and R₂ may be each independently H. That is, the compound represented by Formula I may include 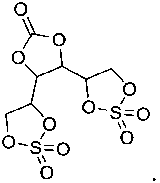 Correspondingly, the compound represented by Formula II may include 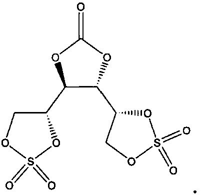 The compound represented by Formula III may include 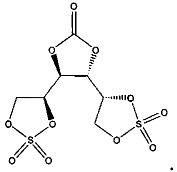 The compound represented by Formula I has strong polarity and weak interaction with other non-polar molecules, resulting in less intermolecular friction and collision. Compared with other sulfate compounds, the compound represented by Formula I has better stability. The compound represented by Formula I may operate stably within a wide voltage range, and possess an excellent cycle life and capacity retention rate. Moreover, this compound may have superior high-temperature performance, maintaining relatively stable electrochemical performance in a high-temperature environment, and thus prolonging a service life of the battery. Further, an oxidation potential and a reduction potential of this compound are both prior to those of a conventional electrolyte solvent. In the battery cycle process, this compound is preferentially oxidized at the positive electrode and reduced at the negative electrode relative to the solvent, facilitating formation of the dense CEI film on the positive electrode and the dense SEI film on the negative electrode. Therefore, the improvement effect of the polycyclic compound on the electrochemical performance of the battery can be further enhanced.

In some specific embodiments of the present disclosure, the polycyclic compound may further include a chlorine-containing organic compound. Based on a mass of the polycyclic compound, a total content of the chlorine-containing organic compound may be less than or equal to 300 ppm, which, for example, may be less than or equal to 300 ppm, less than or equal to 280 ppm, less than or equal to 250 ppm, less than or equal to 220 ppm, less than or equal to 200 ppm, less than or equal to 180 ppm, less than or equal to 150 ppm, less than or equal to 120 ppm, less than or equal to 100 ppm, less than or equal to 80 ppm, less than or equal to 50 ppm, less than or equal to 40 ppm, less than or equal to 30 ppm, less than or equal to 20 ppm, less than or equal to 10 ppm, less than or equal to 5 ppm, and the like. A content of free chloride ions in the electrolyte is strictly controlled, which is generally required to be less than 5 ppm. Free chloride ions not only easily induce corrosion of a positive electrode foil (e.g., an aluminum foil) but also easily lead to dissolution of transition metal ions from a positive electrode sheet of the battery, exerting a significant impact on the battery performance. Currently, those skilled in the art are committed to reducing the content of free chloride ions in the electrolyte. However, in practice, the performance still fails to reach an optimal level. It was found by the inventor of the present disclosure that a conventional method for detecting chloride ions in the electrolyte cannot detect a certain chlorine-containing organic compound with a specific structure. The reason is that these chlorine-containing organic compounds with the specific structure are difficult to lose chlorine under existing test conditions to exist in the form of chloride ions. Therefore, the existing test method cannot detect the chlorine-containing organic compound with the specific structure, enabling those skilled in the art to overlook control of the content of chlorine-containing organic compound. Yet precisely these chlorine-containing organic compounds with the specific structure have a relatively large negative impact on performance of the electrolyte. The reason may be as follows. A battery cell usually contains trace amounts of water and some residual basic substances. If the content of chlorine-containing organic impurities in the electrolyte additive is too high, after the additive is applied in the electrolyte, the chlorine-containing organic impurities are prone to hydrolysis under high-temperature and high-pressure conditions during battery operation, especially under the action of residual basic substances. In this way, an increase in the content of free chlorine in the electrolyte may occur, which not only easily induces the corrosion of the positive electrode foil (e.g., the aluminum foil) but also easily causes the dissolution of transition metal ions from the positive electrode sheet of the battery. Therefore, not only structural stability of the positive electrode active material is reduced but also the dissolved transition metal ions are easily deposit on a surface of a negative electrode, resulting in problems such as continuous decomposition and regeneration of the SEI film, and continuous consumption of active lithium. Both the dissolution of transition metal ions and the decomposition and regeneration of the SEI film may promote decomposition of the electrolyte, which in turn easily leads to battery capacity fading, increased polarization caused by thickening of the SEI film, electrolyte consumption, or blockage of a lithium intercalation/deintercalation channel by a by-product, and an increased short-circuit risk. In this way, low-temperature discharge, high-temperature storage, and cycling performance are significantly affected. Even though a water content in the battery cell is controlled in accordance with relevant standards, the standards impose stricter requirements on free chloride ions than on the water content. That is, even if the water content in the battery cell is not high, under the action of water that exceeds the standard for free chloride ions, once the chlorine-containing organic compound hydrolyzes, the content of free chloride ions may easily exceed the standard, deteriorating the performance. However, based on the conventional synthesis process of the compound represented by Formula I, impurities are inevitably present, making it difficult to obtain a product with high purity and a low impurity content. In particular, an existing synthesis method using sulfonyl chloride as a reaction raw material generally contains a relatively large amount of chlorine-containing organic impurities. The impurity components include, but are not limited to, a chlorine-containing organic compound without cyclic sulfate groups and cyclic carbonate groups, a chlorine-containing organic compound containing only cyclic carbonate groups, a chlorine-containing organic compound containing only cyclic sulfate groups, or a chlorine-containing organic compound containing 1 to 2 cyclic carbonate groups and 1 cyclic sulfate group. These chlorine-containing organic impurities are prone to hydrolysis. When the polycyclic compound is introduced into the electrolyte as the additive, an increase in the content of free chlorine in the electrolyte may occur, which easily leads to not only corrosion of the positive electrode foil but also dissolution of transition metal ions from the active material of the positive electrode sheet. As a result, the improvement effect of the compound represented by Formula I on the electrochemical performance of the battery is reduced. In the present disclosure, by controlling the total content of the chlorine-containing organic compound in the polycyclic compound to be less than or equal to 300 ppm, an increase in the content of free chlorine in the electrolyte caused by introduction of the additive can be effectively reduced. Further, a risk of the positive electrode foil corrosion and transition metal ion dissolution from the positive electrode sheet, which are easily induced by the increased content of free chlorine in the electrolyte, can be reduced. Therefore, a problem of a reduced improvement effect of the compound represented by Formula I on the electrochemical performance of the battery, caused by presence of chlorine-containing organic impurities, can be effectively addressed. This enhances the improvement effect of the polycyclic compound on the electrochemical performance of the battery and achieves enhanced high-temperature performance and cycling performance.

In some specific embodiments of the present disclosure, the chlorine-containing organic compound may include, but is not limited to, the chlorine-containing organic compound without cyclic sulfate groups and cyclic carbonate groups, the chlorine-containing organic compound containing only cyclic carbonate groups, the chlorine-containing organic compound containing only cyclic sulfate groups, or the chlorine-containing organic compound containing 1 to 2 cyclic carbonate groups and 1 cyclic sulfate group. In the synthesis process of the compound represented by Formula I, the chlorine-containing organic compound may be generated due to reasons such as the excessive amount of hexahydric alcohol and insufficient process conditions for the transesterification reaction and the condensation reaction. The several types of chlorine-containing organic impurities mentioned above are common impurities in the target product when the compound represented by Formula I is prepared using the hexahydric alcohol, the carbonate, and the thionyl chloride as the raw material. Reducing the content of the chlorine-containing organic compound is beneficial for further enhancing the improvement effect of the polycyclic compound on the electrochemical performance of the battery.

In some specific embodiments of the present disclosure, based on the mass of the polycyclic compound, the total content of the chlorine-containing organic compound may be less than or equal to 100 ppm, which, for example, may be less than or equal to 100 ppm, less than or equal to 80 ppm, less than or equal to 50 ppm, less than or equal to 30 ppm, or may range from 1 ppm to 99 ppm. Meeting the given range can further reduce the increase in the content of free chlorine in the electrolyte that may be caused by the introduction of the polycyclic compound. It further reduces the risk of positive electrode foil corrosion and transition metal ion dissolution from the positive electrode sheet, which are easily induced by the increased content of free chlorine in the electrolyte. Therefore, the problem of the reduced improvement effect of the compound represented by Formula I on the electrochemical performance of the battery caused by the presence of impurities can be addressed, and thus the improvement effect of the polycyclic compound on the electrochemical performance of the battery can be enhanced. Further, based on the mass of the polycyclic compound, the total content of chlorine-containing organic compound may be less than or equal to 50 ppm. In this way, the problem of the reduced improvement effect of the compound represented by Formula I on the electrochemical performance of the battery caused by the presence of impurities can be further addressed.

In some specific embodiments of the present disclosure, the chlorine-containing organic compound may include one or more of 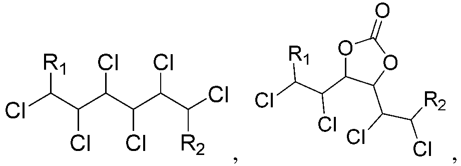 or 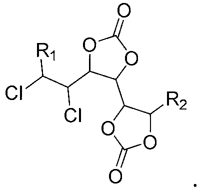 Taking the case where both R₁ and R₂ are H as an example, the chlorine-containing organic compound may include one or more of 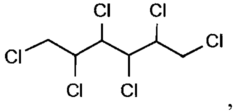 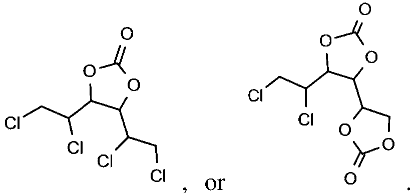 The three types of chlorine-containing organic compounds are common chlorine-containing organic impurities in the target product when the compound represented by Formula I is prepared using the hexahydric alcohol, the carbonate, and the thionyl chloride as the raw material. Moreover, the above-mentioned chlorine-containing organic compounds are prone to hydrolysis to form free chlorine, which may increase the risk of positive electrode foil corrosion and transition metal ion dissolution from the positive electrode sheet. Reducing the content of these three types of chlorine-containing organic compounds is beneficial for further reducing the increase in free chlorine content in the electrolyte that may be caused by the introduction of the polycyclic compound. It lowers the risk of transition metal ion dissolution from the positive electrode sheet, and enhances the improvement effect of the polycyclic compound on the electrochemical performance of the battery. Further, based on the mass of the polycyclic compound, the total content of 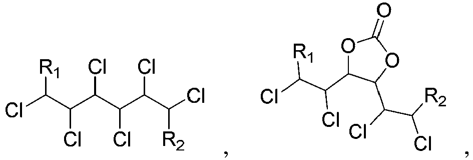 and 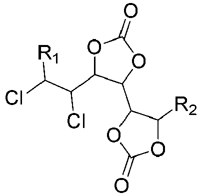 may be less than or equal to 100 ppm, preferably less than or equal to 50 ppm, which, for example, may be less than or equal to 45 ppm, less than or equal to 40 ppm, less than or equal to 35 ppm, less than or equal to 30 ppm, less than or equal to 20 ppm, less than or equal to 10 ppm, or less than or equal to 5 ppm. Therefore, the problem of the reduced improvement effect of the compound represented by Formula I on the electrochemical performance of the battery caused by chlorine-containing organic impurities can be further addressed.

In some specific embodiments of the present disclosure, the polycyclic compound further includes a trans-isomer represented by Formula III. In the compound represented by Formula I, a content of the trans-isomer represented by Formula III is less than or equal to 1%, which, for example, may be 1%, 0.9%, 0.8%, 0.7%, 0.6%, 0.5%, 0.4%, 0.3%, 0.2%, or 0.1%, and the like. Therefore, a problem that the presence of the trans-isomer represented by Formula III reduces the improvement effect of the target product on a side reaction caused by transition metal dissolution can be further avoided. Further, the improvement effect on the electrochemical performance of the battery when the polycyclic compound is introduced into the electrolyte as the additive can be further enhanced, achieving enhanced high-temperature performance and cycling performance. Furthermore, based on the mass of the polycyclic compound, the content of the trans-isomer represented by Formula III may be less than or equal to 0.5%, and optionally less than or equal to 0.1%. A structural formula of the trans-isomer represented by Formula III is as follows:

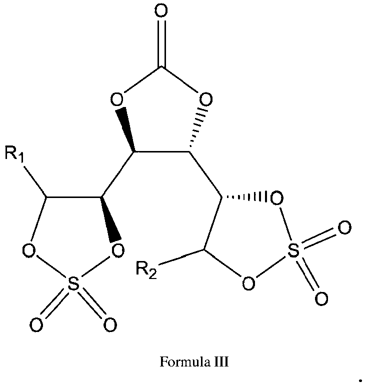

In some specific embodiments of the present disclosure, R₁ and R₂ are each independently selected from any one of H, F, or fluoromethyl. Therefore, not only synthesis of the compound represented by Formula I can be facilitated, but also introduction of a chlorine-containing compound into the additive can be further avoided, reducing the content of free chlorine that may be generated after the electrolyte additive is introduced into the electrolyte. When at least one of R₁ or R₂ is F or fluoromethyl, fluorine substitution or fluoromethyl substitution may be performed on terminal groups of the hexahydric alcohol. The terminal groups have higher reactivity and are more prone to substitution. Then, hexahydric alcohol with fluorine-substituted or fluoromethyl-substituted terminal groups is mixed with raw materials such as the carbonate to perform the transesterification reaction and the subsequent condensation reaction and oxidation reaction.

In some specific embodiments of the present disclosure, the polycyclic compound further includes other organic compounds being selected from one or more of the following 6 compounds: 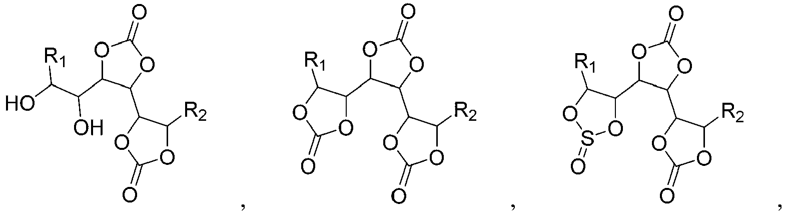 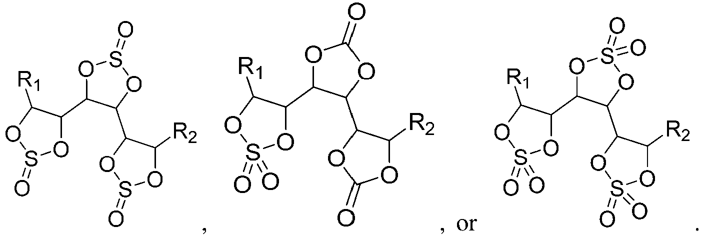 Taking the case where both R₁ and R₂ are H as an example, other organic compounds may be selected from one or more of the following 6 compounds: 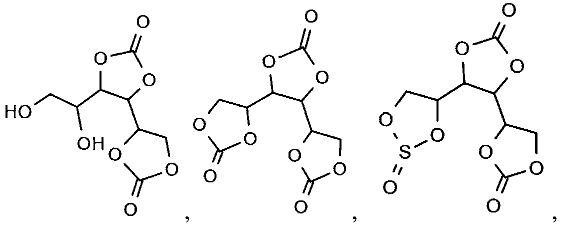 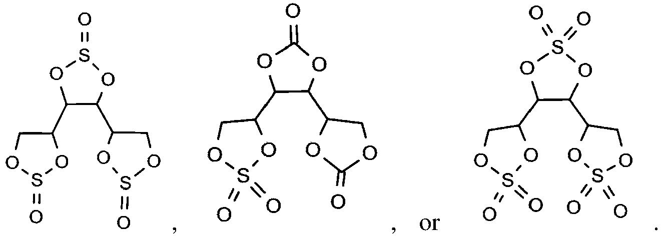 The several compounds mentioned above are also common impurities in the target product when the compound represented by Formula I is prepared using the hexahydric alcohol, the carbonate, and the thionyl chloride as the raw material. Reducing the total content of the other compounds is beneficial for further enhancing the improvement effect of the polycyclic compound on the electrochemical performance of the battery. Based on the mass of the polycyclic compound, a total content of these 6 compounds may be less than or equal to 1000 ppm, which, for example, may be less than or equal to 900 ppm, less than or equal to 800 ppm, less than or equal to 700 ppm, less than or equal to 600 ppm, less than or equal to 500 ppm, less than or equal to 300 ppm, less than or equal to 200 ppm, less than or equal to 100 ppm, less than or equal to 50 ppm, or less than or equal to 30 ppm. Reducing the total content of these 6 compound impurities helps to further lower the risk of the reduced improvement effect of the compound represented by Formula I on the electrochemical performance of the battery due to the presence of impurities. Controlling a total content of impurities to meet the given range can further enhance the improvement effect of the polycyclic compound on the electrochemical performance of the battery. Further, based on the mass of the polycyclic compound, the total content of the 6 compounds may be less than or equal to 200 ppm.

In some specific embodiments of the present disclosure, based on the mass of the polycyclic compound, a content of the compound represented by Formula I may range from 99 wt% to 99.99 wt%, which, for example, may be 99 wt%, 99.1 wt%, 99.5 wt%, 99.8 wt%, 99.9 wt%, 99.95 wt%, or 99.99 wt%. In particular, when the content of the compound represented by Formula I is greater than 99.9 wt%, the improvement effect of the polycyclic compound on the electrochemical performance of the battery can be further enhanced.

In some specific embodiments of the present disclosure, in the compound represented by Formula I, the content of the cis-isomer represented by Formula II may be greater than or equal to 98.9 wt%, which, for example, may be 98.1 wt%, 98.2 wt%, 98.3 wt%, 98.5 wt%, 98.7 wt%, 98.9 wt%, 98.95%, 99%, 99.1 wt%, 99.2 wt%, 99.3 wt%, 99.5 wt%, 99.7%, 99.9 wt%, 99.95 wt%, or 99.99 wt%. In particular, when the content of the cis-isomer represented by Formula II in the compound represented by Formula I is greater than or equal to 98.9 wt%, the improvement effect of the polycyclic compound on the electrochemical performance of the battery can be further enhanced. Further, the content of the cis-isomer represented by Formula II in the compound represented by Formula I may be greater than or equal to 99.9 wt%.

In some specific embodiments of the present disclosure, the content of the cis-isomer represented by Formula II in the compound represented by Formula I may be less than or equal to 99.99 wt%; and/or in the compound represented by Formula I, the content of the trans-isomer represented by Formula III is greater than or equal to 0.01 wt%. Since an addition amount of the compound represented by Formula I in the electrolyte does not exceed 5 wt%, when the content of the cis-isomer represented by Formula II in the compound represented by Formula I is greater than or equal to 99.99 wt%, and the content of the trans-isomer represented by Formula III in the compound represented by Formula I is less than 0.01 wt%, a content of the trans-isomer represented by Formula III in the electrolyte may be less than 5 ppm. Due to a low content in an entire electrolyte system, the trans-isomer represented by Formula III has a very small impact on the effect of the compound represented by Formula I. In this case, even if the content of the cis-isomer represented by Formula II in the compound represented by Formula I is further increased, small improvement in the battery performance may occur. Therefore, based on comprehensive consideration of factors such as performance and a cost, the content of the cis-isomer represented by Formula II in the compound represented by Formula I is further selected to be less than or equal to 99.99 wt%.

Currently, in the common synthesis processes of the compound represented by Formula I, there are problems such as poor selectivity of the target product, a low yield of the target product, and a large amount of impurities generated alongside the target product. In addition, there are also issues including an excessive solvent amount, a cumbersome process, and inconvenience for industrial production. Therefore, it is urgent to develop a method for synthesizing a polycyclic sulfate that features a high conversion rate, a high yield, a relatively simple reaction step, and suitability for industrial production. Also, due to selection and purity of the raw material, different conformations and other impurity components may also exist in the target product. As mentioned earlier, the trans-isomer represented by Formula III cannot effectively eliminate the side reaction caused by the transition metal, which may reduce the improvement effect of the cis-isomer on the battery performance. However, the cis-isomer represented by Formula II has a significant improvement effect on the side reaction caused by transition metal dissolution. Based on the same inventive concept as the first aspect of the present disclosure, it is desired to obtain a polycyclic compound product in which purity of the cis-isomer represented by Formula II in the compound represented by Formula I is greater than or equal to 98 wt%. For this purpose, a method for preparing the polycyclic compound according to a second aspect of the present disclosure is developed. A synthetic route thereof involves forming a substituted or unsubstituted EC (substituted or unsubstituted ethylene carbonate) ring from a substituted or unsubstituted hexahydric alcohol through transesterification, followed by condensation and oxidation. Taking the case where both R₁ and R₂ are H as an example:

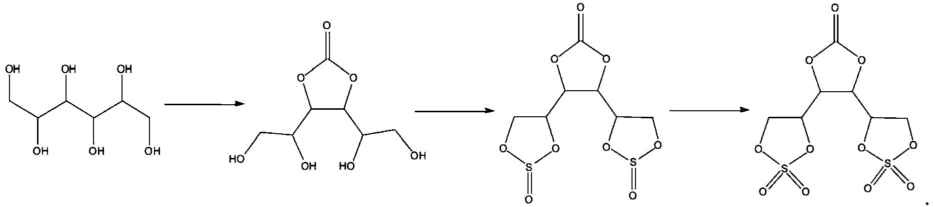

In a second aspect, the present disclosure provides a method for preparing a polycyclic compound. The method includes: (1) mixing a hexahydric alcohol, a monohydric alcohol, a carbonate, and a basic catalyst, and performing a transesterification reaction, to obtain a compound 1; (2) dissolving the compound 1 in a solvent, and performing a condensation reaction with thionyl chloride, to obtain a compound 2; and (3) reacting the compound 2 with an oxidizing agent, to obtain the compound represented by Formula I. Structural formulas of the compound 1 and the compound 2 are represented by 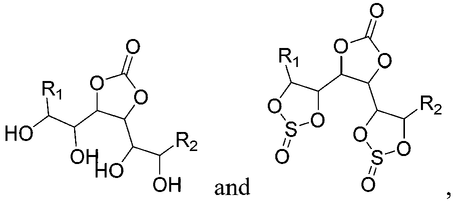 respectively. The hexahydric alcohol includes 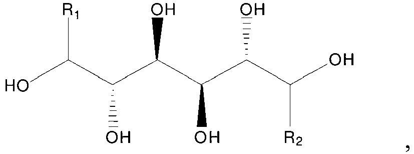 and a mass percentage of 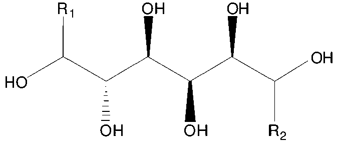 in the hexahydric alcohol is less than or equal to 2%. R₁ and R₂ are each independently selected from any one of H, F, alkyl, or fluoroalkyl.

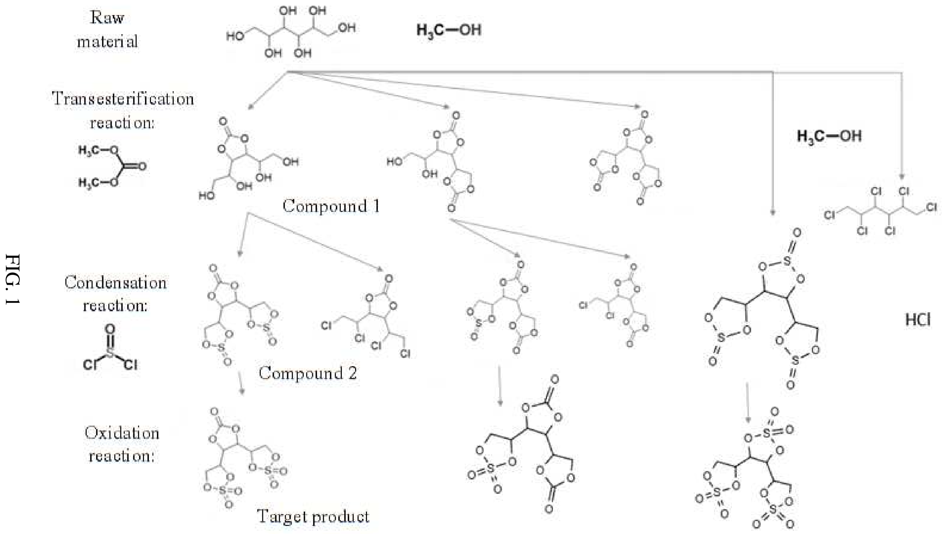

Exemplarily, FIG. 1 shows a flow chart for preparing the target product 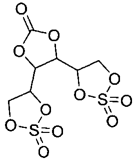 using a mannitol as the hexahydric alcohol, 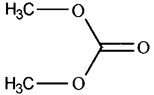 as the carbonate, H₃C-OH as the monohydric alcohol, and the thionyl chloride 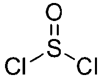 as the raw material. The figure also shows an intermediate product, the target product, and possible impurity components such as the chlorine-containing organic compound and other organic compounds in the preparation process. The mass percentage of 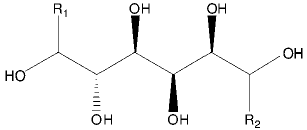 in the hexahydric alcohol may be 2%, or less than or equal to 1.8%, less than or equal to 1.5%, less than or equal to 1.2%, less than or equal to 1%, less than or equal to 0.9%, less than or equal to 0.8%, less than or equal to 0.7%, less than or equal to 0.6%, less than or equal to 0.5%, less than or equal to 0.4%, less than or equal to 0.3%, less than or equal to 0.2%, or less than or equal to 0.1%.

The hexahydric alcohol has high flexibility and a strong intermolecular force. Also, 4 carbon atoms in the hexahydric alcohol are chiral, resulting in multiple possible molecular conformations of the hexahydric alcohol. In the present disclosure, the hexahydric alcohol 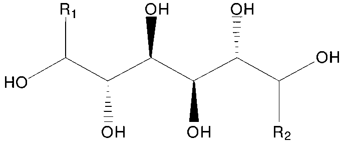 is used as the raw material. However, inevitably some hexahydric alcohols with a structure of 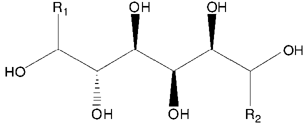 may occur. As an example, the mannitol ( 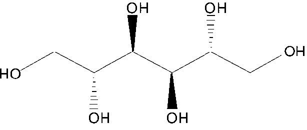 ) is described as the raw material of the hexahydric alcohol. Compared with a sorbitol ( 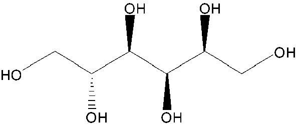 ) and a galactitol ( 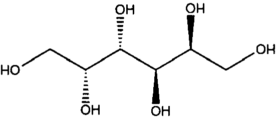 ), using the mannitol as the raw material not only can result in higher selectivity of the target product, but also can make the target product dominated by the cis-isomer represented by Formula II. Taking the mannitol as an example, the current industrial production method of this hexahydric alcohol mainly includes a kelp extraction method and a catalytic hydrogenation method. The catalytic hydrogenation method is a main international production method for mannitol. A main reason is that the kelp purification method involves cumbersome processes for refining and removing impurities such as polysaccharides, with a low yield, a high manufacturing cost, and a raw material source restricted by regions and seasons. Considering the raw material cost, the catalytic hydrogenation method usually uses low-cost sucrose as the raw material, which undergoes hydrolysis, isomerization, and other processes to obtain sucrose, fructose, or glucose-fructose syrup. This product is then used as the raw material for catalytic hydrogenation. However, regardless of the raw material used to produce mannitol, presence of sorbitol is unavoidable. A mannitol product prepared by a current mainstream method contains sorbitol in an amount substantially equivalent to that of mannitol. Also, since mannitol and sorbitol may form a eutectic, separation thereof may be difficult. Purification processes of concentration, cooling crystallization, separation, and drying may increase a content of mannitol. In addition, the more purification cycles are performed, the higher purity of mannitol obtained, and the higher corresponding cost of a mannitol raw material. Therefore, from a cost perspective, except for special fields such as medicine and food where the mannitol has proven effects while sorbitol does not and thus a content of sorbitol in mannitol is controlled, other fields especially the chemical industry do not have special requirements on a content of sorbitol in mannitol to reduce the manufacturing cost. Since those skilled in the art do not recognize an impact of the by-product (such as the trans-isomer represented by Formula III) on performance of the compound represented by Formula I, control of the content of sorbitol in mannitol may often be overlooked. However, in practice, taking R₁ and R₂ as H as an example, in the present disclosure, even if the trans structure formed by reaction of sorbitol undergoes bond rotation, a spatial distance between DTD (i.e., ethylene sulfate) rings on two sides of the trans structure is still larger than that of the cis-structure. Overlap of the DTD rings on two sides is weakened, making it difficult to form a suitable intermediate cage-like structure to capture and stabilize the transition metal. Therefore, based on a special mechanism by which the target product of the present disclosure exerts an effect, in the preparation method of the present disclosure, it is necessary to control the content of the cis-isomer represented by Formula II in the hexahydric alcohol.

In the present disclosure, the hexahydric alcohol 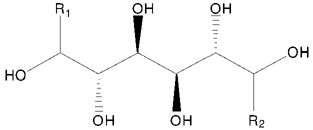 with the cis-structure is used as the raw material. By controlling the mass percentage of 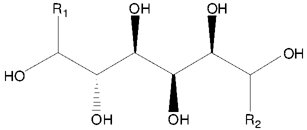 in the hexahydric alcohol to be less than or equal to 2%, the finally prepared compound represented by Formula I can be dominated by the cis-isomer represented by Formula II, which can greatly reduce a content of the trans-isomer represented by Formula III in the final product. That is, generation of a trans-isomer by-product can be inhibited from a perspective of raw material conformation. Therefore, the problem of the reduced improvement effect of the compound represented by Formula I on the electrochemical performance of the battery, caused by the presence of the trans-isomer in the compound represented by Formula I, can be effectively addressed. Therefore, the improvement effect of the prepared polycyclic compound on the electrochemical performance (such as the high-temperature performance and cycling performance) of the battery can be enhanced.

In some specific embodiments of the present disclosure, the transesterification reaction may be performed at a temperature ranging from 65°C to 100°C under a pressure controlled to be less than or equal to 2.5 MP for 0.5 hours to 6 hours. For example, for the transesterification reaction, the temperature may be 65°C, 70°C, 80°C, 90°C, or 100°C,the pressure may be 0.2 MPa, 0.4 MPa, 0.6 MPa, 0.8 MPa, 1 MPa, 1.2 MPa, 1.4 MPa, 1.6 MPa, 1.8 MPa, 2 MPa, 2.2 MPa, or 2.5 MPa, and the duration may be 0.5 h, 1 h, 2 h, 3 h, 4 h, 5 h, or 6 h.

As illustrated in FIG. 1, it should be understood that in the step (1), reactivity of terminal hydroxyl groups (at the 1st position and at the 6th position) of the hexahydric alcohol can be inhibited by using the monohydric alcohol and under the pressure controlled to be less than 2.5 MPa. Subsequently, the reaction temperature may be increased to range from 65°C to 100°C, which is a temperature higher than a boiling point of the monohydric alcohol (among the monohydric alcohols used in the present disclosure, methanol has a lowest boiling point at 65°C) by means of the pressure. Therefore, reactivity of middle hydroxyl groups (at the 2nd position, at the 3rd position, at the 4th position, and at the 5th position) of the hexahydric alcohol can be enhanced, resulting in the middle hydroxyl groups having significantly higher reactivity than the terminal hydroxyl groups. Also, under the conditions of heating and pressurization, a product formed by the reaction of the hydroxyl groups at the 3rd position and at the 4th position (i.e., the compound 1) have better symmetry and stability compared to a product formed by the reaction of the hydroxyl groups at the 2nd position and at the 3rd position or a product formed by the reaction of hydroxyl groups at the 4th position and at the 5th position. Therefore, under these conditions, selectivity for the compound 1 and a conversion efficiency of the compound 1 in the transesterification reaction can be improved, yielding an intermediate product containing the compound 1 with a high conversion rate and high selectivity. Compared with a conventional process for synthesizing the target product represented by Formula I, where selectivity is low and the by-product is easily generated, the process of the step (1) in the present disclosure can effectively reduce a content of residual hexahydric alcohol in the transesterification reaction product, and a content of impurities 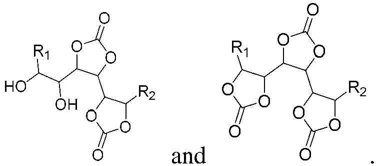 Further, the generation of impurities 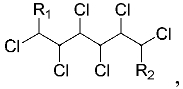 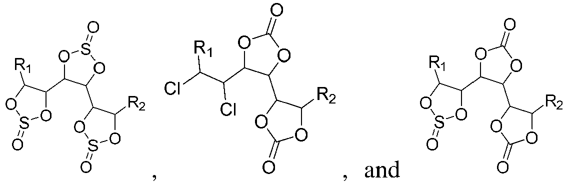 that may exist in a condensation reaction product of the step (2) can be reduced, and a content of impurities 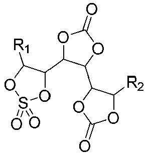 and 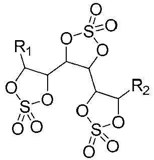 that may be generated in the subsequent oxidation reaction can be reduced.

In the present disclosure, since the monohydric alcohol is used as the reaction raw material, limited by the boiling point of the monohydric alcohol (among the monohydric alcohols used in the present disclosure, methanol has the lowest boiling point of 65°C), the monohydric alcohol may evaporate and reflux when a temperature of the monohydric alcohol reaches a boiling point under a normal pressure or a negative pressure in a reaction system of the present disclosure. During reflux, a solution temperature and a vapor temperature may adjust each other continuously, and finally reach a dynamic equilibrium. It was found by the inventor that under this dynamic equilibrium, the temperature of the reaction system may stabilize around the boiling point of the monohydric alcohol, making it difficult to increase the temperature of the reaction system, and thus limiting the reaction rate. However, the pressurization method in the present disclosure can not only cooperate with the monohydric alcohol to improve reaction selectivity, but also increase the boiling point of the monohydric alcohol, leading to a proper increase in the reaction temperature and ultimately improving the reaction rate. An excessively low pressure in the transesterification reaction (e.g., under the negative pressure) may lead to massive removal of the monohydric alcohol in the reaction system, which is not conducive to improving selectivity of the transesterification reaction and inhibiting generation of the impurity 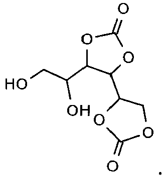 An excessively high pressure in the transesterification reaction may, as the reaction proceeds, easily hinder progress of the transesterification reaction if there is too much methanol in the system, resulting in a reduced conversion rate and an increased residual raw material. In the present disclosure, by controlling the temperature, pressure, and duration of the transesterification reaction within the above ranges, both a relatively high raw material conversion rate and the suitable transesterification reaction rate can be balanced, thereby obtaining the compound 1 with the high selectivity, the high yield, and the low impurity content.

In addition, in the step (1), no additional organic solvent is used in the transesterification reaction. Carbonate and monohydric alcohol serve as both reactants and solvents, making the process not only environmentally friendly but also easy to operate. Problems existing in the synthesis of the compound represented by Formula I, such as a low reaction conversion rate, cumbersome reaction steps, high impurity generation, and inconvenience for industrial production, can be effectively improved.

Therefore, in some embodiments of the present disclosure, the polycyclic compound can be prepared by combining selection of a hexahydric alcohol raw material and controlling the above-mentioned transesterification reaction conditions. As a result, not only the high selectivity and high conversion rate for the compound represented by Formula I can be achieved, but also the generated compound represented by Formula I can be dominated by the cis-isomer, with a low content of trans-isomer and impurities. In this way, the problems existing in the synthesis of the compound represented by Formula I, such as the low reaction conversion rate, cumbersome reaction steps, high generation of the trans-isomer by-product and impurities, and inconvenience for industrial production, can be effectively addressed. Also, obtaining the compound represented by Formula I with high purity can be facilitated, and a mass percentage of a target product cis-isomer represented by Formula II in the compound represented by Formula I is greater than or equal to 98 wt%. Using the prepared polycyclic compound in the electrolyte can not only effectively reduce the content of the trans-isomer represented by Formula III in the electrolyte, but also reduce a content of a chlorine-containing organic compound that may be introduced into the electrolyte and a content of free chlorine that may be generated by the chlorine-containing organic compound. Further, the risk of positive electrode foil corrosion and transition metal ion dissolution from the positive electrode sheet, which are easily induced by the increased free chlorine content in the electrolyte, can be reduced. Therefore, a problem of a reduced improvement effect of the compound represented by Formula I on the electrochemical performance of the battery caused by presence of trans-isomer and chlorine-containing organic impurities in the compound represented by Formula I can be effectively addressed. Therefore, the improvement effect of the prepared polycyclic compound on the electrochemical performance (such as the high-temperature performance and the cycling performance) of the battery can be enhanced.

Further, in some specific embodiments of the present disclosure, the transesterification reaction may be performed at a temperature ranging from 70°C to 90°C and a pressure ranging from 0.5 MPa to 2.2 MPa. More preferably, the transesterification reaction may be performed at a temperature ranging from 75°C to 85°C and a pressure ranging from 1 MPa to 2 MPa. Therefore, further balancing the relatively high raw material conversion rate and the suitable transesterification reaction rate can be facilitated, thereby obtaining the compound 1 with the high selectivity, the high yield, and the low impurity content.

It should be noted that in some specific embodiments of the present disclosure, a source of the hexahydric alcohol 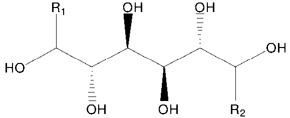 (i.e., a target hexahydric alcohol) with a mass percentage of 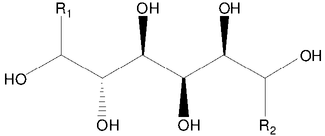 less than or equal to 2% is not particularly limited, which may be flexibly selected by those skilled in the art as desired. For example, the hexahydric alcohol may be obtained by optimizing a preparation process of the hexahydric alcohol to improve purity of the target hexahydric alcohol, or purifying an existing target hexahydric alcohol to reduce a content of 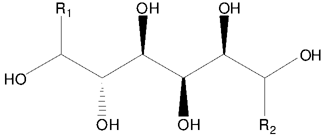 in the target hexahydric alcohol, or purchasing from a manufacturer capable of manufacturing the target hexahydric alcohol. Exemplarily, separation of 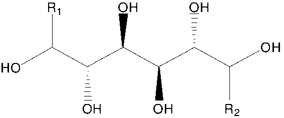 and 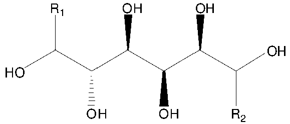 may be achieved based on a solubility difference at different temperatures, reducing a content of 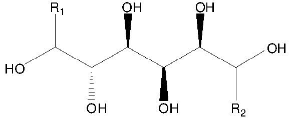 in the target hexahydric alcohol 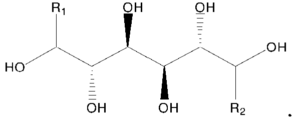 Taking the mannitol as an example, the mannitol may be purified by recrystallization based on a solubility difference between the mannitol and the sorbitol at different temperatures. For example, the existing mannitol may be prepared into an aqueous solution with a mass concentration of 50%, concentrated at 80°C, then cooled for crystallization, and the crystal is separated, dried, and finally packaged at a low temperature. The more purification times indicate that the purity of the obtained mannitol is higher, and the content of sorbitol is lower.

In some specific embodiments of the present disclosure, a high-performance liquid chromatography column may be used to test a content of 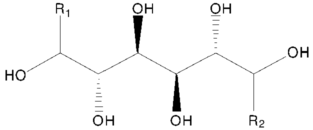 and 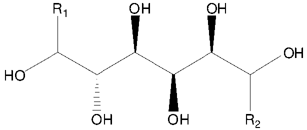 in the hexahydric alcohol. Taking the mannitol and the sorbitol as examples, 1.0 g of the sample may be weighed and placed in a clean beaker, and 100 mL of a mixed solution of ultrapure water: acetonitrile=1:1 (v/v) is added and stirred uniformly. The above solution is filtered through an aqueous filter membrane with a 0.22 µm pore size, and a 20 µL liquid phase syringe is used to sample for high-performance liquid chromatography detection. A model of the high-performance liquid chromatograph is Agilent 1290 Infinity II (equipped with an ELSD detector), and the chromatographic column is Agilent Poroshell 120 EC-C18 with a specification of 4.6×150 mm×2.7 µm. A column temperature is 30 °C. An injection volume is 10 µL. A mobile phase A is 0.1% trifluoroacetic acid aqueous solution (0.1% v/v). A mobile phase B is acetonitrile. A flow rate is 1 mL/min, and gradient elution is used. In the obtained chromatogram, a mass percentage of mannitol and a mass percentage of sorbitol in the raw material are quantified by an area normalization method.

In some specific embodiments of the present disclosure, as illustrated in FIG. 1, it should be understood that in the step (1), a molar ratio of the carbonate to the hexahydric alcohol may range from 3:1 to 5:1, which, for example, may be 3/1, 3.5/1, 4/1, 4.5/1, or 5/1. Based on an amount of the hexahydric alcohol, appropriately increasing an amount of the carbonate is conducive to accelerating progress of the transesterification reaction, improving a conversion rate of the raw material, and reducing a content of an unreacted hexahydric alcohol in the intermediate product, thereby helping to reduce the content of impurities 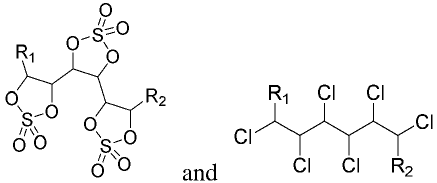 in the final product. In the present disclosure, by controlling the amount of the carbonate to meet the given range, the transesterification reaction can have an appropriate reaction rate. In this way, not only a utilization rate of the raw material and a yield of the compound 1 can be improved, reducing the content of impurities 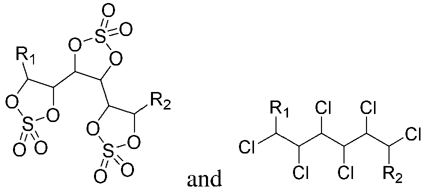 in the final product, but also the risk that excessive addition of the carbonate leads to an excessively fast transesterification reaction, which causes the terminal hydroxyl groups of the hexahydric alcohol to react and generate impurities 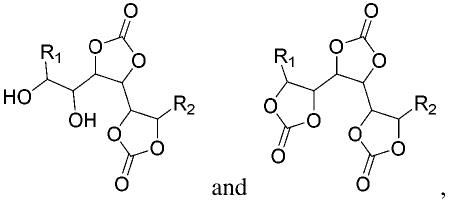 can be reduced. Further, the content of impurities 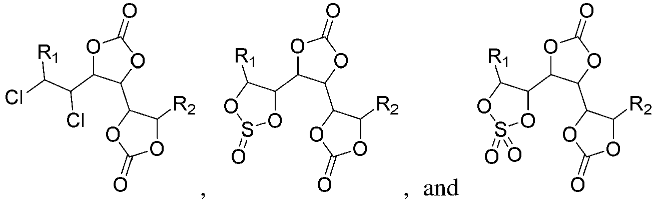 that may be formed subsequently can be further reduced.

In some specific embodiments of the present disclosure, as illustrated in FIG. 1, it should be understood that in the step (1), a molar ratio of the monohydric alcohol to the hexahydric alcohol may range from 10:1 to 20:1, which, for example, may be 10/1, 12/1, 14/1, 16/1, 18/1, 20/1, etc. Further preferably, a molar ratio of the monohydric alcohol to the mannitol may range from (12:1) to (18:1). Even more preferably, the molar ratio of the monohydric alcohol to the mannitol may range from (14:1) to (16:1). In the present disclosure, the monohydric alcohol serves as the solvent on the one hand, and an addition amount of the monohydric alcohol usually only needs to meet a requirement of completely dissolving the reaction raw materials mannitol and carbonate. Generally, when the molar ratio of the monohydric alcohol to the mannitol is greater than or equal to 10, the dissolution requirement may be met. The related art mentions that excessive addition of solvent methanol is acceptable for dissolving the reaction raw material. However, actually it was found by the inventor that the content of monohydric alcohol also affects selectivity of the transesterification reaction. If the content of monohydric alcohol is too high (for example, when the molar ratio of the monohydric alcohol to the mannitol is greater than 20:1), the selectivity of the transesterification reaction may be reduced to a certain extent. Controlling the amount of the monohydric alcohol relative to the mannitol to meet the above range can not only meet a demand of using the monohydric alcohol as the solvent to dissolve the reaction raw material, but also reduce a risk that an excessive monohydric alcohol is likely to exert a strong inhibitory effect on the transesterification reaction, which is conducive to smooth progress of the transesterification reaction. In addition, the transesterification reaction can have an appropriate reaction rate. In this way, an issue that an insufficient monohydric alcohol is not conducive to improving the selectivity of the transesterification reaction, which may cause the terminal hydroxyl groups of the mannitol to react and generate impurities 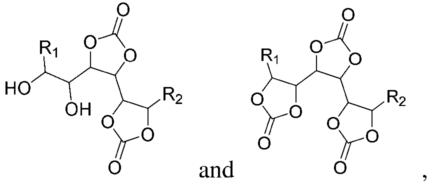 can be mitigated. Further, the content of impurities 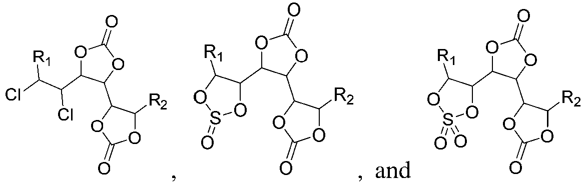 that may be formed subsequently can be further reduced.

In some specific embodiments of the present disclosure, in the step (1), based on a mass of the hexahydric alcohol, an amount of the basic catalyst may range from 0.01 wt% to 5 wt%, which, for example, may be 0.01 wt%, 0.1 wt%, 0.5 wt%, 1 wt%, 2 wt%, 3 wt%, 4 wt%, 5 wt%, etc. Controlling the amount of the basic catalyst within the above range is not only conducive to the smooth progress of the transesterification reaction, but also can reduce a risk of an increased amount of the thionyl chloride and thus excessive sulfur dioxide generation in the subsequent condensation reaction caused by an excessive amount of the basic catalyst.

In some specific embodiments of the present disclosure, in the step (1), based on a mass of the mannitol, an amount of the basic catalyst may range from 0.02 wt% to 0.5 wt%. Further preferably, based on the mass of the mannitol, the amount of the basic catalyst may range from 0.02 wt% to 0.1 wt%. In the present disclosure, to facilitate industrialization, a post-treatment process of each reaction step is expected to be simplified. Since the basic catalyst is used in the transesterification reaction in the step (1), the reaction system tends to be alkaline after the reaction. If no neutralization or purification process is performed, the basic catalyst may enter the condensation reaction process, consume the thionyl chloride in the second step, and affect the condensation reaction. However, removing the basic catalyst may complicate the post-treatment process of the step (1), which may require adding acidic substances such as oxalic acid to adjust a pH value, followed by post-treatment steps such as filtration and recrystallization. In the present disclosure, by further reducing the amount of the basic catalyst, the post-treatment steps such as pH adjustment, filtration, and recrystallization after the step (1) can be simplified. However, reducing a catalyst content may easily affect the reaction rate. In the present disclosure, heating and pressurization are used to make up for a defect caused by a reduced catalyst amount, achieving a goal of simplifying the post-treatment process. On this basis, by further regulating relative amounts of the mannitol, the monohydric alcohol, and the carbonate, and making the reaction temperature and a pressure condition to meet the above range, the defect caused by the reduced catalyst amount can be further compensated, achieving the goal of simplifying the post-treatment process.

In some specific embodiments of the present disclosure, in the step (1), the carbonate may include, but is not limited to, one or more of dimethyl carbonate, diethyl carbonate, ethyl methyl carbonate, or diphenyl carbonate.

In some specific embodiments of the present disclosure, in the step (1), the monohydric alcohol may include, but is not limited to, one or more of methanol, ethanol, propanol, isopropanol, butanol, or tert-butanol.

In some specific embodiments of the present disclosure, in the step (1), the basic catalyst may include, but is not limited to, one or more of sodium hydroxide, potassium hydroxide, potassium carbonate, sodium carbonate, lithium carbonate, sodium methoxide, sodium ethoxide, sodium tert-butoxide, potassium tert-butoxide, triethylamine, or pyridine.

In some specific embodiments of the present disclosure, a mass percentage of 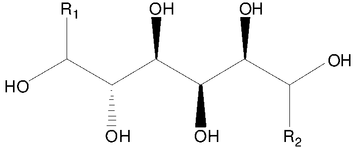 in the hexahydric alcohol may be less than or equal to 1%. Further, the mass percentage of 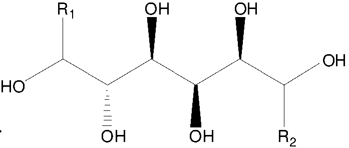 in the hexahydric alcohol may be less than or equal to 0.5%. Still further, the mass percentage of 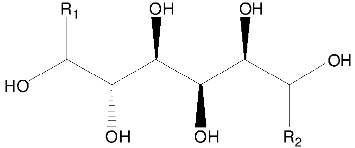 in the hexahydric alcohol may be less than or equal to 0.1%. Therefore, a content of a trans-isomer by-product represented by Formula III in the final product can be further reduced.

In some specific embodiments of the present disclosure, the mass percentage of 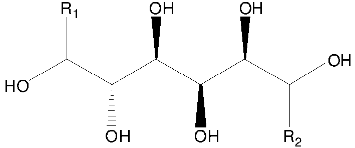 in the hexahydric alcohol is greater than or equal to 0.01%. Taking the mannitol as an example, the industrial production method of this hexahydric alcohol mainly includes the kelp extraction method and the catalytic hydrogenation method. The catalytic hydrogenation method is the main international production method for the mannitol. The main reason is that the kelp purification method involves cumbersome processes for refining and removing impurities such as polysaccharides, with low yield, high manufacturing cost, and raw material source restricted by regions and seasons. Considering the raw material cost, the catalytic hydrogenation method usually uses low-cost sucrose as the raw material, which undergoes hydrolysis, isomerization, and other processes to obtain sucrose, fructose, or glucose-fructose syrup. This product is then used as the raw material for catalytic hydrogenation. However, regardless of the raw material used to produce the mannitol, presence of sorbitol is unavoidable. The mannitol product prepared by the current mainstream method contains sorbitol in the amount substantially equivalent to that of mannitol. Also, since mannitol and sorbitol may form the eutectic, separation thereof may be difficult. Purification processes of concentration, cooling crystallization, separation, and drying may increase the content of mannitol. In addition, the more purification cycles are performed, the higher purity of mannitol obtained, and the higher corresponding cost of the mannitol raw material. According to experimental results, after the content of the sorbitol in the mannitol is lower than 0.01 wt%, when the product prepared using this mannitol as the raw material is applied in the battery, improvement in the battery performance with a decrease in the content of the sorbitol in the mannitol is small and almost negligible. A possible reason is that a content of the compound represented by Formula 1 in the present disclosure when used in the electrolyte does not exceed 5 wt%. When the content of the sorbitol in the mannitol is lower than 0.01 wt%, a content of the trans-isomer represented by Formula III in the product of the compound represented by Formula 1 may be lower than 5 ppm in the electrolyte. Due to a low content in the entire electrolyte system, the trans-isomer represented by Formula III has little impact on the effect of the compound represented by Formula 1. In this case, even if the content of the trans-isomer represented by Formula III in the compound represented by Formula 1 is further reduced (i.e., the content of the sorbitol in the mannitol is reduced), the improvement in the battery performance may be small. Based on this, on the premise of meeting the requirement of improving the battery performance, and considering both performance and the cost, it is more preferable that the mass percentage of 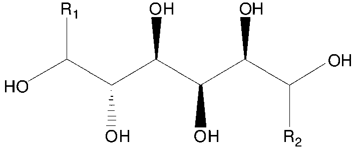 in the hexahydric alcohol is greater than or equal to 0.01%. In some specific embodiments of the present disclosure, the hexahydric alcohol may be the mannitol. A mass percentage of the sorbitol in the mannitol may be less than or equal to 2%, preferably less than or equal to 1%, more preferably less than or equal to 0.5%, and even more preferably less than or equal to 0.1%. Therefore, a target product dominated by 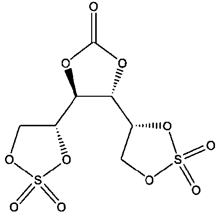 can be obtained.

In some specific embodiments of the present disclosure, in the step (1), subsequent to completion of the transesterification reaction, the method further includes: performing a reduced-pressure treatment to remove a low-boiling component. Therefore, the purity of the compound represented by Formula I in the finally prepared product can be improved, reducing the content of impurities. As some specific examples, the reduced-pressure treatment may be a reduced-pressure distillation treatment. Optionally, a pressure of the reduced-pressure treatment may range from -90 kPa to -80 kPa (that is, 90 kPa to 80 kPa lower than a standard atmospheric pressure), which, for example, may be -90 kPa, -85 kPa, -80 kPa, etc. A temperature of the reduced-pressure treatment may range from 30°C to 60°C, which, for example, may be 35°C, 40°C, 45°C, 50°C, 55°C, etc. In this way, the low-boiling component can be further removed.

In some specific embodiments of the present disclosure, in the step (2), subsequent to completion of the condensation reaction, the method further includes: performing a reflux deacidification treatment. Therefore, the residual HCl generated by the condensation reaction can be reduced, reducing the content of free chlorine that may be introduced into the electrolyte subsequently. In some specific embodiments, a temperature of the reflux deacidification treatment may range from 40°C to 80°C, which, for example, may be 40°C, 50°C, 60°C, 70°C, or 80°C. In some specific embodiments, a duration of the reflux deacidification treatment may range from 10 minutes to 50 minutes, which, for example, may be 15 minutes, 20 minutes, 30 minutes, 40 minutes, or 50 minutes. Meeting the reflux deacidification treatment conditions is conducive to further reducing the residual HCl generated by the condensation reaction.

In some specific embodiments of the present disclosure, as illustrated in FIG. 1, in the step (2), subsequent to dissolving the compound 1 in the solvent, the condensation reaction is performed by adding thionyl chloride dropwise. By using this method to slowly add the thionyl chloride into the mixed solution to react with the compound 1, a side reaction that may be caused by a relative excess of thionyl chloride can be significantly reduced, effectively inhibiting generation of the impurity

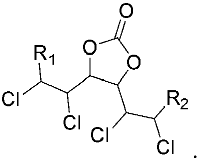

In some specific embodiments of the present disclosure, in the step (2), the condensation reaction may be performed at a temperature ranging from 20°C to 100°C, which, for example, may range from 20°C to 60°C, or may be 20°C, 25°C, 30°C, 50°C, 60°C, 80°C, or 100°C. Controlling the temperature of the condensation reaction within the given range is not only beneficial to reducing a risk of increased chlorine-containing impurities (such as 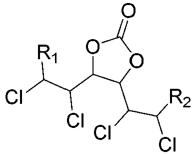 ) caused by increased reactivity of the thionyl chloride due to an excessively high reaction temperature, but also helps to lower risks of a reduced reaction rate, a prolonged reaction duration, and a decreased manufacturing efficiency caused by an excessively low reaction temperature, achieving a good balance between the reaction efficiency and the purity of the final product. In addition, a duration of the condensation reaction may range from 1 hour to 4 hours, for example, 1 hour, 2 hours, 3 hours, or 4 hours. The duration refers to a period from a start of adding the thionyl chloride to an end of the condensation reaction. The duration may be adaptively adjusted according to an addition duration of the thionyl chloride, enabling the reaction to continue for a period of time after completion of thionyl chloride addition to ensure complete progress of the condensation reaction.

In some specific embodiments of the present disclosure, in the step (2), a molar ratio of the compound 1 to the thionyl chloride may be 1:(2 to 3), which, for example, may be 1/2, 1/2.2, 1/2.5, 1/2.8, 1/3, etc. An addition duration for the thionyl chloride may range from 0.5 hours to 2 hours, which, for example, may be 0.5 hours, 0.8 hours, 1 hour, 1.5 hours, or 2 hours, etc. Controlling the addition amount of thionyl chloride within the given range is conducive to reducing a risk of a decreased conversion rate of the mannitol due to a relatively insufficient thionyl chloride, which may result in incomplete reaction of hydroxyl groups and further lead to reduced purity and yield of the compound 2 and the target product represented by Formula I. Also, it contributes to lower the risk of the increased chlorine-containing impurities when the amount of thionyl chloride is relatively large. Further, making the addition duration of the thionyl chloride meet the given range can achieve an appropriate addition rate of the thionyl chloride, which can not only improve the reaction rate but also reduce the risk of the increased chlorine-containing impurities caused by an excessively fast addition rate of the thionyl chloride. Therefore, the reaction rate can be balanced, and the purity of the compound 2 and the final product can be improved, reducing the impurity content.

In some specific embodiments of the present disclosure, in the step (2), the solvent may include, but is not limited to, one or more of an ether solvent, N,N-dimethylformamide (DMF), N,N-dimethylacetamide (DMAC), tetrahydrofuran, or an ester solvent.

In some specific embodiments of the present disclosure, in the step (3), the oxidizing agent may be provided as an aqueous solution. Subsequent to reacting the compound 2 with the oxidizing agent, the method further includes: performing a heating treatment on a reaction product to remove water. As a specific example, when the solvent added in the step (2) can azeotrope with water, the heating treatment may be an azeotropic treatment of the reaction product. The azeotropic treatment is also beneficial to further reducing metal cation impurities and free chloride ions that may be introduced into the final product during reaction.

In some specific embodiments of the present disclosure, in the step (3), after the oxidation reaction is completed, the reaction product may be washed with water to remove inorganic salts. Specifically, an organic phase in the reaction product may be separated, and deionized water may be added for extraction and stratification. After repeating this operation several times, the solvent is removed to obtain the compound represented by Formula I. In some specific embodiments, removal of the solvent may be achieved by the reduced-pressure treatment. The pressure of the reduced-pressure treatment may range from -90 kPa to -80 kPa (i.e., 90 kPa to 80 kPa lower than the standard atmospheric pressure), which, for example, may be -90 kPa, -85 kPa, -80 kPa, etc. The temperature of the reduced-pressure treatment may range from 30°C to 60°C, which, for example, may be 35°C, 40°C, 45°C, 50°C, 55°C, etc. Therefore, further removing a solvent component can be facilitated.

In some specific embodiments of the present disclosure, in the step (3), a specific type of the oxidizing agent is not particularly limited, which may be flexibly selected by those skilled in the art as desired. For example, the oxidizing agent may include, but is not limited to, one or more of hydrogen peroxide, sodium hypochlorite, or sodium periodate. For example, the oxidizing agent may be the sodium hypochlorite. For another example, the oxidizing agent may be the hydrogen peroxide. Choosing the hydrogen peroxide can further reduce chlorine-containing impurities that may be introduced into the final product, and generated moisture can be removed by the heating treatment.

In some specific embodiments of the present disclosure, in the step (3), the oxidizing agent may include the sodium hypochlorite and/or the sodium periodate. A molar ratio of the compound 2 to the oxidizing agent may be 1:(2 to 2.5), which, for example, may be 1/2, 1/2.1, 1/2.2, 1/2.3, 1/2.4, or 1/2.5. Controlling the content of the oxidizing agent within the given range is beneficial to fully oxidizing the compound 2, improving the yield and purity of the target product represented by Formula I, and also reducing chlorine-containing impurities and/or inorganic salt impurities that may be introduced when the amount of the oxidizing agent is relatively large.

In some specific embodiments of the present disclosure, in the step (3), the oxidizing agent may include the sodium hypochlorite and/or the sodium periodate. The oxidizing agent may be provided as the aqueous solution. Before reacting the compound 2 with the oxidizing agent, the condensation reaction product is dissolved in a non-aqueous solvent, and then the oxidizing agent is added for the oxidation reaction. After the oxidation reaction, the method further includes: (4-1) performing the heating treatment on the reaction product to remove water; (4-2) after water removal, inorganic salts precipitate out, and the inorganic salts are removed by filtration. Therefore, not only water and inorganic salts from the final product can be removed, for example, a content of the sodium hypochlorite or a content of the sodium periodate can be reduced to several tens or even several ppm, but also a loss of the target product caused by a water washing process can be avoided.

It should be noted that the method for preparing the polycyclic compound in the second aspect of the present disclosure and the polycyclic compound in the first aspect of the present disclosure are provided based on the same inventive concept. Characteristics and effects described for the polycyclic compound in the first aspect of the present disclosure are also applicable to the method for preparing the polycyclic compound in the second aspect of the present disclosure, and will not be repeated herein.

In a third aspect, the present disclosure provides a polycyclic compound prepared by the above-mentioned method for preparing the polycyclic compound. The prepared polycyclic compound includes the compound represented by Formula I. In the compound represented by Formula I, the content of the cis-isomer represented by Formula II is greater than or equal to 98 wt%. Characteristics and effects described for the above-mentioned method for preparing the polycyclic compound are also applicable to this polycyclic compound, and will not be repeated herein.

In a fourth aspect, the present disclosure provides use of the above-mentioned polycyclic compound and the above-mentioned method for preparing the polycyclic compound in the field of electrolyte and battery. Characteristics and effects described for the above-mentioned polycyclic compound and the above-mentioned method for preparing the polycyclic compound are also applicable to this use, and will not be repeated herein.

In a fifth aspect, the present disclosure provides an electrolyte. The electrolyte includes: an electrolyte additive. The electrolyte additive includes the above-mentioned polycyclic compound or the polycyclic compound prepared by the above-mentioned method for preparing the polycyclic compound. It should be noted that characteristics and effects described for the above-mentioned polycyclic compound and the above-mentioned method for preparing the polycyclic compound are also applicable to this electrolyte, and will not be repeated herein. In general, applying this electrolyte to the battery can improve the high-temperature performance and cycling performance of the battery well.

Typically, the electrolyte further includes electrolyte salt and an organic solvent. Optionally, the electrolyte may be used in the lithium battery. Taking the lithium battery as an example, the electrolyte salt may be lithium salt. The organic solvent is a main component of the electrolyte and an important carrier for ion transmission. The organic solvent may have high lithium salt solubility, making the electrolyte have high ionic conductivity. After the lithium salt dissolves in the organic solvent, lithium ions can be released. The lithium ions form a solvated structure with the organic solvent, which is conducive to rapid migration of lithium ions.

In some specific embodiments of the present disclosure, based on a total mass of the electrolyte, a content of the electrolyte additive may range from 0.1 wt% to 5 wt%, which, for example, may be 0.5 wt%, 1 wt%, 1.5 wt%, 2 wt%, 2.5 wt%, 3 wt%, 3.5 wt%, 4 wt%, 4.5 wt%, 5 wt%, etc. Controlling the content within this range is conducive to controlling the content of the trans-isomer (by-product) represented by Formula III in the electrolyte below 500 ppm. Therefore, when the electrolyte is applied to the battery, a good improvement effect can be exerted on the high-temperature performance and cycling performance of the battery.

In some specific embodiments of the present disclosure, the organic solvent may include a cyclic compound or a linear compound. The cyclic compound may include, but is not limited to, at least one of propylene carbonate, ethylene carbonate, γ-butyrolactone, sulfolane, or fluoroethylene carbonate. The linear compound may include, but is not limited to, at least one of dimethyl carbonate, ethyl methyl carbonate, diethyl carbonate, methyl propyl carbonate, ethyl acetate, propyl propionate, ethyl propionate, propyl acetate, methyl propionate, 1,1,2,2-tetrafluoroethyl-2,2,3,3-tetrafluoropropyl ether, or 2,2-difluoroethyl acetate. After dissolving the lithium salt, the organic solvent within the given range can enable the electrolyte to have high ionic conductivity, further improving the electrochemical performance of the battery.

In some specific embodiments of the present disclosure, specific composition of the organic solvent may be flexibly adjusted according to actual conditions such as the battery system. For example, as some specific examples, the organic solvent may include ethylene carbonate (EC), ethyl methyl carbonate (EMC), and dimethyl carbonate (DMC) in a mass ratio of 1:(0.5 to 2):(0.5 to 2). This organic solvent may be applied to a battery system using a layered transition metal oxide as a positive electrode active material (for example, a positive electrode active material with a chemical formula of LiₐNi_{b}Co_{c}M1_{d}M2ₑO_{f}R_{g}, where 1≤a≤1.2, 0.6≤b≤1, 0≤c≤1, 0≤d≤1, 0≤e≤0.2, b+c+d+e=1, 1≤f≤2, 0≤g≤1, f+g=2; M1 includes Mn and/or Al; M2 includes at least one of Zr, Zn, Cu, Cr, Mg, Fe, V, Ti, Sr, Sb, Y, W, or Nb; and R includes at least one of N, F, S, or Cl).

In some specific embodiments of the present disclosure, the lithium salt may include a main salt. The main salt may include lithium hexafluorophosphate (LiPF₆) and/or lithium bis(fluorosulfonyl)imide (LiFSI). Optionally, a mass percentage of the lithium hexafluorophosphate in the electrolyte may range from 4% to 18%; and/or, a mass percentage of the lithium bis(fluorosulfonyl)imide in the electrolyte may range from 0% to 10%. A cost of lithium salt accounts for a relatively high proportion of a cost of the electrolyte. Controlling the content of the lithium salt in the electrolyte within the given range is not only conducive to full dissolution of the lithium salt in the organic solvent, but also helps the electrolyte to have both high ionic conductivity and a low manufacturing cost. Further optionally, a mass percentage of the organic solvent in the electrolyte may range from 10% to 90%.

In some specific embodiments of the present disclosure, in addition to the above components, the electrolyte may further optionally include a small amount of other conventional additives or auxiliaries that can improve certain performances of the battery, which may be selected by those skilled in the art as desired, and will not be repeated herein.

In a sixth aspect, the present disclosure provides a battery. The battery includes the above-mentioned electrolyte. It should be noted that characteristics and effects described for the above-mentioned electrolyte of the present disclosure are also applicable to this battery, and will not be repeated herein. In general, this battery has good high-temperature performance and cycling performance. In addition, it should be noted that a type of the battery is not particularly limited, which can be flexibly selected by those skilled in the art as desired. For example, the battery may be a secondary battery.

Typically, the battery includes the positive electrode sheet, a negative electrode sheet, the electrolyte, and a separator. During charging and discharging of the battery, active ions intercalate and deintercalate back and forth between the positive electrode sheet and the negative electrode sheet. The electrolyte provides conduction for ions between the positive electrode sheet and the negative electrode sheet. The separator is disposed between the positive electrode sheet and the negative electrode sheet, mainly to prevent a short circuit between a positive electrode and a negative electrode, and also to make ions pass through.

In some embodiments of the present disclosure, the positive electrode sheet may include a positive electrode current collector and a positive electrode active material layer disposed on a surface of the positive electrode current collector. The positive electrode active material layer includes the positive electrode active material, a conductive agent, and a binder. The positive electrode current collector may include a metal foil or a composite positive electrode current collector. For example, the metal foil may be the aluminum foil. The composite positive electrode current collector may include a polymer material substrate and a metal layer formed on at least one surface of the polymer material substrate. For example, the composite positive electrode current collector may be formed by forming a metal material (aluminum, aluminum alloy, nickel, nickel alloy, etc.) at a polymer material substrate (such as polypropylene (PP), polyethylene terephthalate (PET), polybutylene terephthalate (PBT), etc.). The positive electrode active material includes a lithium iron phosphate material, a nickel-cobalt-manganese ternary material, a lithium manganese iron phosphate material, a lithium manganese oxide material, a lithium nickel manganese oxide material, a lithium cobalt oxide material, a nickel-cobalt-aluminum material, a lithium-rich manganese oxide material, or a sodium-ion battery material, and may also be other positive electrode active materials commonly used in the field. The conductive agent and the binder may be conventional materials in the field.

In some embodiments of the present disclosure, the negative electrode sheet may include a negative electrode current collector and a negative electrode active material layer disposed on a surface of the negative electrode current collector. The negative electrode active material layer may include a negative electrode active material, the conductive agent, and the binder. The negative electrode current collector may be the metal foil or a composite current collector. For example, the metal foil may be a copper foil. The composite current collector may include the polymer material substrate and the metal layer formed on the at least one surface of the polymer material substrate. For example, the composite current collector may be formed by forming a metal material (copper, copper alloy, nickel, nickel alloy, etc.) at the polymer material substrate (such as polypropylene (PP), polyethylene terephthalate (PET), polybutylene terephthalate (PBT), etc.). The negative electrode active material may include a negative electrode active material commonly used in the field (for example, may include, but is not limited to, at least one of graphite, a silicon-carbon composite material, a silicon material, lithium metal, lithium titanate, or other commonly used negative electrode active materials). The conductive agent and the binder may be conventional materials in the field.

In some embodiments of the present disclosure, the separator may be a separator known in the art that may be used in the battery and stable to the electrolyte used. A material of the separator may include, but is not limited to, at least one of polyolefin, aromatic polyamide, polytetrafluoroethylene, or polyethersulfone. For example, the separator may be a polyethylene separator, a polypropylene separator, a PE ceramic-coated separator, etc., which can be flexibly selected as desired.

The embodiments of the present disclosure are described in detail below. It should be noted that the embodiments described below are exemplary and are only intended to explain the present disclosure, rather than limit the present disclosure. In addition, unless otherwise specified, all reagents used in the following embodiments are either commercially available or can be synthesized according to the methods described herein or by known procedures. Any reaction conditions not explicitly stated are also readily accessible to those skilled in the art.

### Example 1

### 1. Preparation of Polycyclic Compound

(i) Mannitol, dimethyl carbonate, methanol, and sodium hydroxide were placed into a reaction kettle. The reaction was performed at 1 MPa and 75°C for 1 hour. A low-boiling material was removed under a reduced pressure of (-85±5) kPa at 50°C, yielding a dry product dominated by the compound 1. A structural formula of the compound 1 was 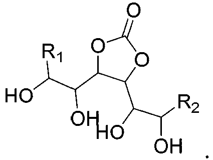 In this example, the structural formula was 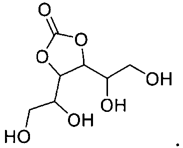 The content of sorbitol in mannitol accounted for 2 wt% of a total mass.
(ii) The dry product was dissolved in dichloromethane (a mass ratio of the dichloromethane to the dry product was 5:2). At a room temperature, the thionyl chloride was added dropwise to the dry product solution, with a dropping duration being 1.5 hours. The mixture was stirred for 3 hours. A molar ratio of 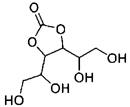 to the thionyl chloride in the dry product was 1:2.1. Reflux deacidification was performed at 40°C for 30 minutes to obtain a reaction product. The reaction product contained the compound 2. A structural formula of the compound 2 was 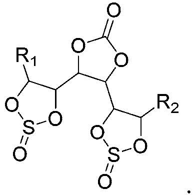 In this example, the structural formula was 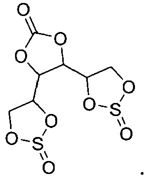
(iii) A sodium hypochlorite aqueous solution with a concentration of 12% was added to the reaction product from the step (ii) for the oxidation reaction. Layer separation was performed. The organic phase was collected. The organic phase was washed with deionized water, followed by extraction and layer separation. This operation was repeated 3 times. The solvent was removed under the reduced pressure of (-85±5) kPa at 30°C, obtaining the final product 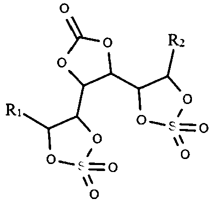 represented by Formula I, which in this example was 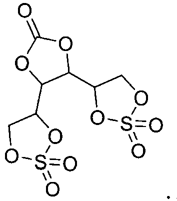 A molar ratio of the sodium hypochlorite to the compound 2 was 2:1.

### 2. Preparation of Positive Electrode Sheet

A positive electrode active material LiNi_{0.5}Co_{0.2}Mn_{0.3}O₂ (NCM523), acetylene black, carbon nanotube, and polyvinylidene fluoride (PVDF) were dispersed in a solvent NMP at a mass ratio of 96.3:2:0.5:1.2, resulting in a positive electrode active material layer slurry. The positive electrode active material layer slurry was uniformly coated at two sides of a positive electrode current collector aluminum foil. After drying, rolling, baking, slitting, and spot-welding of a tab, the positive electrode sheet was obtained. A total thickness of the positive electrode sheet was 116 µm, with a compaction density of 3.3 g/cm³ and a double-sided areal density of 33 mg/cm² (excluding the foil).

### 3. Preparation of Negative Electrode Sheet

Artificial graphite, a conductive agent Super P (conductive carbon black), a binder styrene-butadiene rubber, and a dispersant CMC (sodium carboxymethyl cellulose) were dispersed in the deionized water at a mass ratio of 95:1.5:2:1.5. The mixture was stirred uniformly to obtain a negative electrode active material layer slurry. The negative electrode active material layer slurry was uniformly coated at two sides of a negative electrode current collector copper foil. After drying, rolling, baking, slitting, and spot-welding of the tab, the negative electrode sheet was obtained. A total thickness of the negative electrode sheet was 134 µm, with a compaction density of 1.6 g/cm³ and a double-sided areal density of 20 mg/cm² (excluding the foil).

### 4. Preparation of Electrolyte

The electrolyte included, by the mass percentage, a main lithium salt, the organic solvent, and the additive. The main lithium salt contained lithium hexafluorophosphate (LiPF₆) with a mass percentage of 12.5%. The organic solvent was composed of ethylene carbonate (EC), ethyl methyl carbonate (EMC), and diethyl carbonate (DEC) at a mass ratio of EC:EMC:DEC=3:5:2. The final product prepared in the step (i) was used as the electrolyte additive. A mass percentage of the electrolyte additive prepared in the step (i) in the electrolyte was 0.5%, which was supplemented with the solvent to reach 100% of the total mass of the electrolyte.

### 5. Preparation of Lithium-Ion Battery

The prepared positive electrode sheet, the prepared negative electrode sheet, and a ceramic separator with a thickness of 12 µm were stacked in sequence, with the separator placed between the positive electrode sheet and the negative electrode sheet. After winding, a wound body was flattened and placed into an aluminum-plastic film packaging bag. Baking at a vacuum condition was conducted at 75°C for 48 hours to obtain a battery cell to be filled with the electrolyte. Subsequently, the above-mentioned electrolyte was injected into the battery cell in a glove box. After encapsulation, formation, aging, and capacity grading, the preparation of the lithium-ion battery was completed, with a designed capacity of 1700 mAh.

### Example 2 to Example 26 and Comparative Example 1 to Comparative Example 8

Differences between Example 2 to Example 26, Comparative Example 1 to Comparative Example 8, and Example 1 were detailed in Table 1 to Table 2.

A structure, purity, cis-isomer/trans-isomer composition, content of chlorine-containing organic impurities of the polycyclic compound obtained in Examples 1 to 26 and Comparative Examples 1 to 8, and the cycling performance of the battery were tested. Test methods were as follows. Test results were shown in Table 2.
(1) The content of sorbitol in a mannitol raw material was tested by high-performance liquid chromatography. The polycyclic compound final product obtained was subjected to NMR testing using a nuclear magnetic resonance (NMR) spectrometer to determine a chemical structure of the final product. The polycyclic compound final product obtained was subjected to liquid chromatography testing using a high-performance liquid chromatograph to conduct quantitative analysis of a component of the final product. A content of the chlorine-containing organic compound was analyzed by the high-performance liquid chromatography. A content of the cis-isomer target product in the final product was tested by the high-performance liquid chromatography.
① A specific method using high-performance liquid chromatography for testing mannitol and sorbitol was as follows:
1) Reagents and Instruments for testing:
a) acetonitrile (HPLC grade);
b) trifluoroacetic acid (AR or GR grade);
c) deionized water (prepared by a water purification system);
d) chromatographic column: AQ C-18, 4.6×250 mm. 5 µm;
e) electronic balance: precision of 0.00001 g;
f) high-performance liquid chromatograph (equipped with an ELSD detector): Agilent 1260 Infinity II.

2) Test Conditions:
a) Mobile phases:
Mobile phase A: 0.1% trifluoroacetic acid+95% water+5% acetonitrile solution (0.1% v/v). Prepared by precisely transferring 1 mL of trifluoroacetic acid into 1 L of acetonitrile-water mixed solution, followed by mixing and sonication.
Mobile phase B: 95% acetonitrile+5% water (v/v).
Diluent: acetonitrile.
A gradient elution program was as shown in Table 1.

**[Table 1]**

| Time (min) | A% | B% |
|---|---|---|
| 0.0 | 10 | 90 |
| 10.0 | 90 | 10 |
| 18.0 | 90 | 10 |

b) flow rate: 1.0 mL/min.
c) injection volume: 5 µL.
d) column temperature: 30°C.
e) acquisition time: 16 min.
3) Test Procedures:
Sample preparation: in the glove box, acetonitrile was added to a sample vial to dissolve the sample. The sample vial was washed with the acetonitrile three times. The solution was poured into a 50 mL volumetric flask, and then acetonitrile was added for volume fixing. The mixture was shaken well, filtered through a 0.45 µm filter into an ampoule, and tested on the instrument. For a peak result of the test sample, all peaks except those in a blank spectrum were integrated. Purity thereof was calculated by the area normalization method. An arithmetic mean was taken as a determination result.

In addition, 6 replicate samples were tested to calculate the purity of the sample via the area normalization and reproducibility RSD%. Samples at 0 h and 24 h were evaluated, with 1 parallel sample tested at 24 h. For each test sample, a total of: 6 replicates+2 samples at 24 h=8 test samples were prepared.

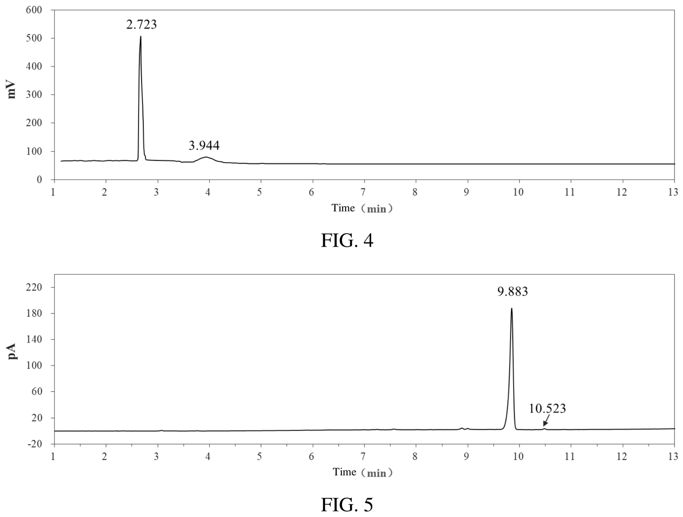

To clearly show a peak position of sorbitol in the spectrum, high-performance liquid chromatography detection was performed using crude mannitol as an example. A detection result was shown in FIG. 4.
② A specific method using high-performance liquid chromatography for testing the target product and isomers was as follows:
1) Reagents and Instruments for testing:
a) acetonitrile (HPLC grade);
b) trifluoroacetic acid (AR or GR grade);
c) deionized water (prepared by the water purification system);
d) chromatographic column: AQ C-18, 4.6×250 mm. 5 µm;
e) electronic balance: precision of 0.00001 g;
f) high-performance liquid chromatograph (equipped with the CAD detector): Thermo Scientific U3000.

2) Test Conditions:
a) Mobile phases:
Mobile phase A: 0.1% trifluoroacetic acid+95% water+5% acetonitrile solution (0.1% v/v). Prepared by precisely transferring 1 mL of trifluoroacetic acid into 1 L of acetonitrile-water mixed solution, followed by mixing and sonication.
Mobile phase B: 95% acetonitrile + 5% water (v/v).
Diluent: acetonitrile.
The gradient elution program was as shown in Table 2.

**[Table 2]**

| Time (min) | A% | B% |
|---|---|---|
| 0.0 | 90 | 10 |
| 10.0 | 10 | 90 |
| 12.0 | 10 | 90 |
| 12.5 | 90 | 10 |
| 15.0 | 90 | 10 |

b) flow rate: 1.0 mL/min.
c) injection volume: 5 µL.
d) column temperature: 30°C.
e) acquisition time: 16 min.
3) Test Procedures:
Sample preparation: in the glove box, the acetonitrile was added to the sample vial to dissolve the sample. The vial was washed with the acetonitrile three times. The solution was poured into the 50 mL volumetric flask, and then acetonitrile was added for volume fixing. The mixture was shaken well, filtered through the 0.45 µm filter into the ampoule, and tested on the instrument. For the peak result of the test sample, all peaks except those in the blank spectrum were integrated. Purity thereof was calculated by the area normalization method. The arithmetic mean was taken as the determination result.

In addition, 6 replicate samples were tested to calculate the purity of the sample via the area normalization and the reproducibility RSD%. Samples at 0 h and 24 h were evaluated, with 1 parallel sample tested at 24 h. For each test sample, a total of: 6 replicates+2 samples at 24 h=8 test samples were prepared.

The high-performance liquid chromatography detection was performed using the polycyclic compound obtained in Example 1 as an example. A detection result was shown in FIG. 5.
(2) Room-Temperature Cycling Performance Test: after formation, the battery was charged at a 3 C constant current to 4.35 V at 25°C, and then charged at a 4.35 V constant voltage until a cut-off current of 0.05 C was reached. The battery was then discharged at 1C to 2.75 V. This charge-discharge cycle was repeated 800 times. A capacity retention rate was calculated as a ratio of a discharge capacity at the 800th cycle to that at the 1st cycle.
(3) High-Temperature Cycling Performance Test: after formation, the battery was charged at the 1 C constant current to 4.35 V at 45°C, and then charged at the 4.35 V constant voltage until the cut-off current of 0.05 C was reached. The battery was then discharged at 1 C to 2.75 V. This charge-discharge cycle was repeated 500 times. The capacity retention rate was calculated as a ratio of a discharge capacity at the 500th cycle to that at the 1st cycle.
(4) High-Temperature Storage Performance Test: at 25°C, the battery was charged at the 1C constant current to 4.35 V, and then charged at the 4.35 V constant voltage until the cut-off current of 0.05 C was reached. The battery was then discharged at 1C to 2.75 V, and a discharge capacity thereof was recorded as C₁. The battery was then stored at 60°C for 14 days, after which the battery was discharged at the 1 C constant current, and a discharge capacity was recorded as C₂. A capacity retention rate at 60°C was calculated as (C₂/C₁)×100%.

### Example 27

Preparation of the polycyclic compound was identical to that in Example 9. Example 27 differed from Example 9 mainly in preparation of the negative electrode sheet, the positive electrode sheet, the electrolyte, and the lithium battery. Also, the test conditions for the relevant tests were different. Details were as follows.

### 1. Preparation of Positive Electrode Sheet

A positive electrode active material LiFePO₄ (LFP), the acetylene black, the carbon nanotube, polyvinylidene fluoride (PVDF), and a lithium supplement agent LFO were dispersed in the solvent NMP at a mass ratio of 93.5:2:0.5:2:2, resulting in the positive electrode active material layer slurry. The positive electrode active material layer slurry was uniformly coated at two sides of the positive electrode current collector aluminum foil. After drying, rolling, baking, slitting, and spot-welding of the tab, the positive electrode sheet was obtained. A total thickness of the positive electrode sheet was 198 µm, with a compaction density of 2.2 g/cm³ and a double-sided areal density of 40 mg/cm² (excluding the foil).

### 2. Preparation of Negative Electrode Sheet

The artificial graphite, the conductive agent Super P (conductive carbon black), the binder styrene-butadiene rubber, and the dispersant CMC (sodium carboxymethyl cellulose) were dispersed in the deionized water at a mass ratio of 95:1.5:2:1.5. The mixture was stirred uniformly to obtain the negative electrode active material layer slurry. The negative electrode active material layer slurry was uniformly coated at two sides of the negative electrode current collector copper foil. After drying, rolling, baking, slitting, and spot-welding of the tab, the negative electrode sheet was obtained. A total thickness of the negative electrode sheet was 129 µm, with a compaction density of 1.5 g/cm³ and a double-sided areal density of 18 mg/cm² (excluding the foil).

### 3. Preparation of Electrolyte

The electrolyte included, by the mass percentage, the main lithium salt, the organic solvent, and the additive. The main lithium salt contained the lithium hexafluorophosphate (LiPF₆) with the mass percentage of 12.5%. The organic solvent was composed of ethylene carbonate (EC), propylene carbonate (PC), ethyl methyl carbonate (EMC), and diethyl carbonate (DEC) at a mass ratio of EC:PC:EMC:DMC = 2:1:6:1. A conventional additive VC was added at 2.5%. The final product prepared in the step (i) was used as the electrolyte additive. A mass percentage of the electrolyte additive prepared in the step (i) in the electrolyte was 0.5%, which was supplemented with the solvent to reach 100% of the total mass of the electrolyte.

### 4. Preparation of Lithium-ion Battery

The prepared positive electrode sheet, the prepared negative electrode sheet, and the ceramic separator with the thickness of 12 µm were stacked in sequence, with the separator placed between the positive electrode sheet and the negative electrode sheet. After winding, the wound body was flattened and placed into the aluminum-plastic film packaging bag. Baking at a vacuum condition was conducted at 75°C for 48 hours to obtain the battery cell to be filled with the electrolyte. Subsequently, the above-mentioned electrolyte was injected into the battery cell in the glove box. After encapsulation, formation, aging, and capacity grading, the preparation of the lithium-ion battery was completed, with a designed capacity of 1500 mAh.

### 5. Test Conditions

(1) Room-Temperature Cycling Performance Test: after formation, the battery was charged at the 1C constant current to 3.65 V at 25°C, and then charged at the 3.65 V constant voltage until the cut-off current of 0.05 C was reached. The battery was then discharged at 1C to 2.0 V. This charge-discharge cycle was repeated 5000 times. A capacity retention rate was calculated as a ratio of a discharge capacity at the 5000th cycle to that at the 1st cycle.
(3) High-Temperature Cycling Performance Test: after formation, the battery was charged at the 1C constant current to 3.65 V at 45°C, and then charged at the 3.65 V constant voltage until the cut-off current of 0.05 C was reached. The battery was then discharged at 1C to 2.0 V. This charge-discharge cycle was repeated 2000 times. The capacity retention rate was calculated as a ratio of a discharge capacity at the 2000th cycle to that at the 1st cycle.
(4) High-Temperature Storage Performance Test: at 25°C, the battery was charged at the 1C constant current to 3.65 V, and then charged at the 3.65 V constant voltage until the cut-off current of 0.05 C was reached. The battery was then discharged at 1C to 2.0 V, and a discharge capacity thereof was recorded as C₁. The battery was then stored at 60°C for 90 days, after which the battery was discharged at 1C constant current, and a discharge capacity was recorded as C₂. A capacity retention rate at 60°C was calculated as (C₂/C₁)×100%.

### Example 28

Preparation of the polycyclic compound was identical to that in Example 9. Example 28 differed from Example 9 mainly in the preparation of the negative electrode sheet, the positive electrode sheet, the electrolyte, and the lithium battery. Also, the test conditions for the relevant tests were different. Details were as follows.

### 1. Preparation of Positive Electrode Sheet

A positive electrode active material LiMnFePO₄ (LMFP), the acetylene black, the carbon nanotube, and the polyvinylidene fluoride (PVDF) were dispersed in the solvent NMP at a mass ratio of 94.1:2:0.5:3.4, resulting in the positive electrode active material layer slurry. The positive electrode active material layer slurry was uniformly coated at two sides of the positive electrode current collector aluminum foil. After drying, rolling, baking, slitting, and spot-welding of the tab, the positive electrode sheet was obtained. A total thickness of the positive electrode sheet was 168 µm, with a compaction density of 2.3 g/cm³ and a double-sided areal density of 35 mg/cm² (excluding the foil).

### 2. Preparation of Negative Electrode Sheet

The artificial graphite, the conductive agent Super P (conductive carbon black), the binder styrene-butadiene rubber, and the dispersant CMC (sodium carboxymethyl cellulose) were dispersed in the deionized water at a mass ratio of 95:1.5:2:1.5. The mixture was stirred uniformly to obtain the negative electrode active material layer slurry. The negative electrode active material layer slurry was uniformly coated at two sides of the negative electrode current collector copper foil. After drying, rolling, baking, slitting, and spot-welding of the tab, the negative electrode sheet was obtained. A total thickness of the negative electrode sheet was 112 µm, with a compaction density of 1.6 g/cm³ and a double-sided areal density of 16.4 mg/cm² (excluding the foil).

### 3. Preparation of Electrolyte

The electrolyte included, by the mass percentage, the main lithium salt, the organic solvent, and the additive. The main lithium salt contained the lithium hexafluorophosphate (LiPF₆) with the mass percentage of 12.5%. The organic solvent was composed of ethylene carbonate (EC), propylene carbonate (PC), ethyl methyl carbonate (EMC), and diethyl carbonate (DEC) at a mass ratio of EC:PC:EMC:DMC = 2:1:5:2. A conventional additive VC was added at 1.7%. The final product prepared in the step (i) was used as the electrolyte additive. A mass percentage of the electrolyte additive prepared in the step (i) in the electrolyte was 0.5%, which was supplemented with the solvent to reach 100% of the total mass of the electrolyte.

### 4. Cell Testing

(1) Room-Temperature Cycling Performance Test: after formation, the battery was charged at the 3C constant current to 4.25 V at 25°C, and then charged at the 4.25 V constant voltage until the cut-off current of 0.05 C was reached. The battery was then discharged at 1 C to a voltage of 2.5 V. This charge-discharge cycle was repeated 800 times. A capacity retention rate was calculated as a ratio of a discharge capacity at the 800th cycle to that at the 1st cycle.
(2) High-Temperature Cycling Performance Test: after formation, the battery was charged at the 1 C constant current to 4.25 V at 45°C, and then charged at the 4.25 V constant voltage until the cut-off current of 0.05 C was reached. The battery was then discharged at 1C to a voltage of 2.5 V. This charge-discharge cycle was repeated 500 times. The capacity retention rate was calculated as a ratio of a discharge capacity at the 500th cycle to that at the 1st cycle.
(3) High-Temperature Storage Performance Test: at 25°C, the battery was charged at the 1C constant current to 4.25 V, and then charged at the 4.25 V constant voltage until the cut-off current of 0.05 C was reached. The battery was then discharged at 1C to 2.5 V, and a discharge capacity thereof was recorded as C₁. The battery was then stored at 60°C for 60 days, after which the battery was discharged at the 1C constant current, and a discharge capacity was recorded as C₂. A capacity retention rate at 60°C was calculated as (C₂/C₁)×100%.

### Comparative Example 9

Comparative Example 9 differed from Example 27 in that the content of sorbitol in the hexahydric alcohol used as the raw material for preparing the polycyclic compound was 99 wt%, while all other preparation methods and test methods were identical.

### Comparative Example 10

Comparative Example 10 differed from Example 28 in that the content of sorbitol in the hexahydric alcohol used as the raw material for preparing the polycyclic compound was 99 wt%, while all other preparation methods and test methods were identical.

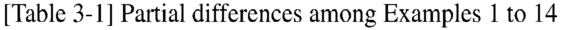

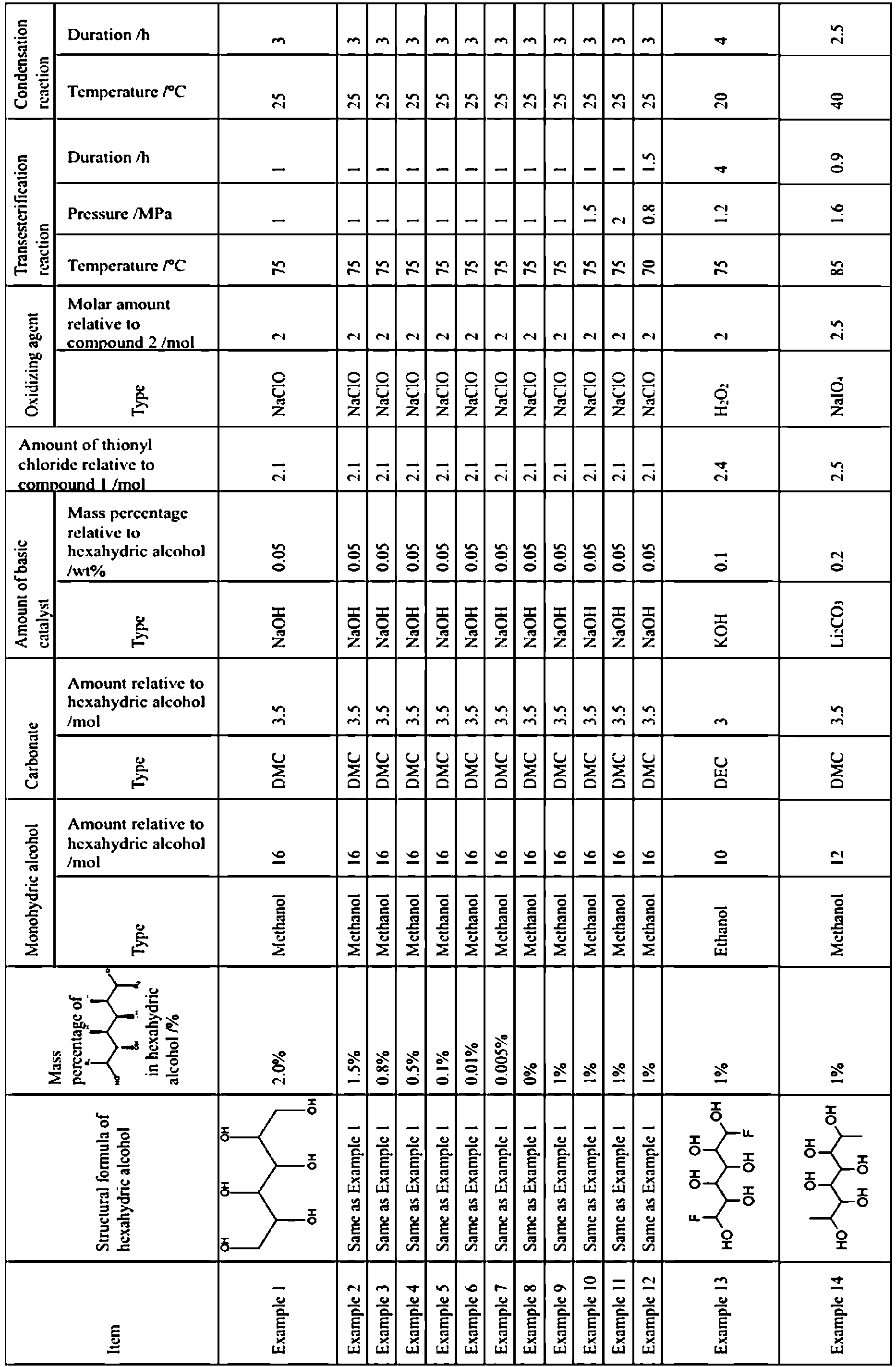

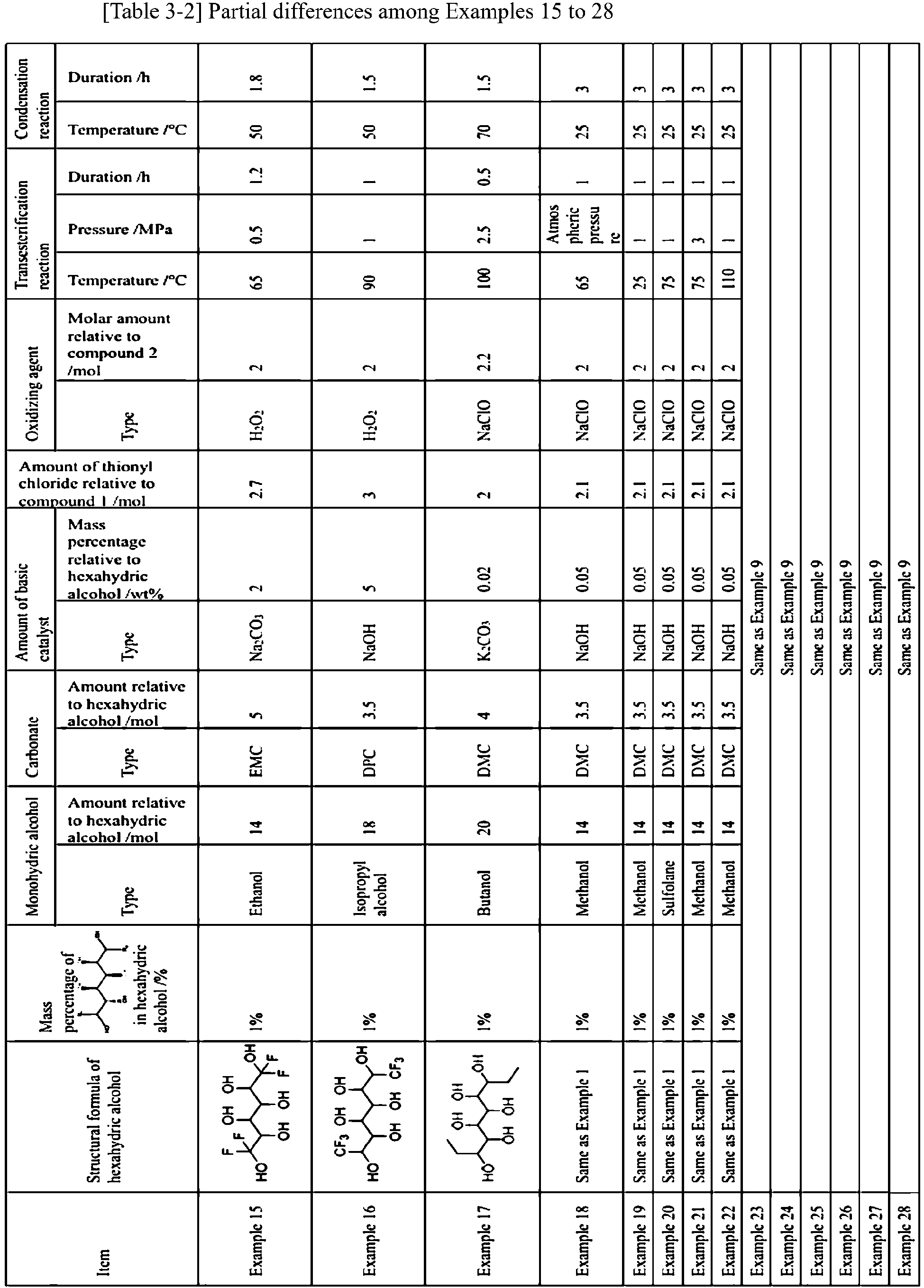

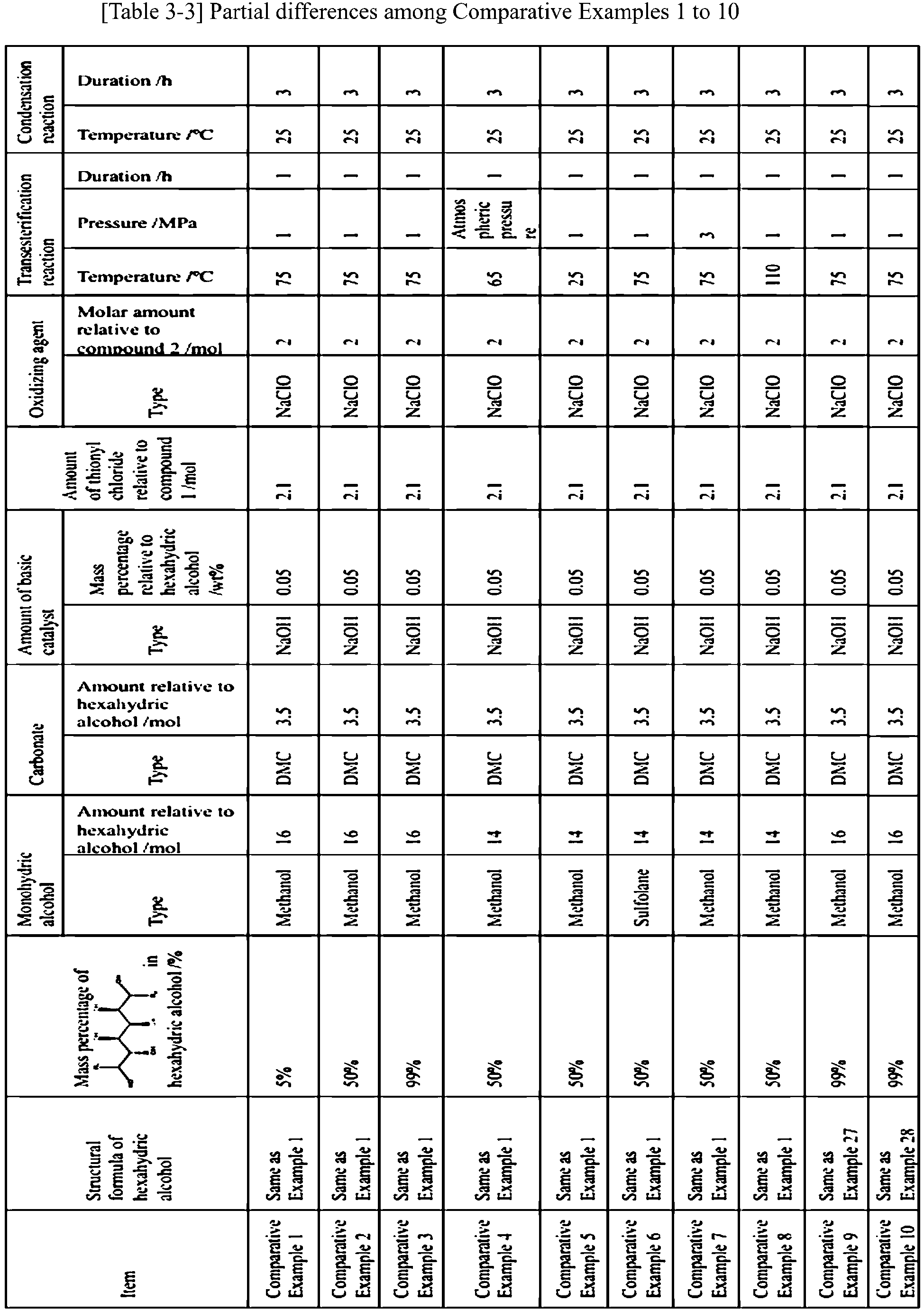

Note: in Table 3-1, Table 3-2, and Table 3-3, the hexahydric alcohol in the examples was dominated by

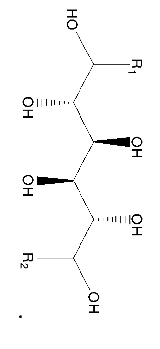

**[Table 4-1] Partial differences and test results of Examples 1 to 12**

| Item | Final product | | | | | | Mass percentage of final product introduced as additive in electrolyte /% | Cycling capacity retention rate at 25°C /% | Cycling capacity retention rate at 45°C /% | Storage capacity retention rate at 60°C /% |
|---|---|---|---|---|---|---|---|---|---|---|
| | Specific structural formula of product represented by Formula I | Yield /% | Purity /% | Content percentage of cis-isomer represented by Formula II in product /% | Content percentage of trans-isomer represented by Formula III in product /% | Total content of chlorine-containing organic compound / ppm | | | | |
| Example 1 | 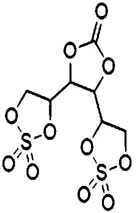 | 91 | 99.97 | 98.2 | 1.8 | 67 | 0.5 | 86.5 | 84.6 | 82.2 |
| Example 2 | Same as Example 1 | 91 | 99.97 | 98.7 | 1.3 | 66 | 0.5 | 87.0 | 85.2 | 83.0 |
| Example 3 | Same as Example 1 | 92 | 99.98 | 99.3 | 0.7 | 67 | 0.5 | 87.2 | 86.0 | 84.1 |
| Example 4 | Same as Example 1 | 93 | 99.98 | 99.5 | 0.5 | 65 | 0.5 | 87.3 | 86.2 | 84.7 |
| Example 5 | Same as Example 1 | 93 | 99.98 | 99.9 | 0.1 | 66 | 0.5 | 87.6 | 86.8 | 85.0 |
| Example 6 | Same as Example 1 | 93 | 99.98 | 100 | N.D | 65 | 0.5 | 87.7 | 86.9 | 85.3 |
| Example 7 | Same as Example 1 | 93 | 99.98 | 100 | N.D | 65 | 0.5 | 87.7 | 86.9 | 85.3 |
| Example 8 | Same as Example 1 | 93 | 99.98 | 100 | N.D | 65 | 0.5 | 87.7 | 86.9 | 85.3 |
| Example 9 | Same as Example 1 | 92 | 99.98 | 99.1 | 0.9 | 68 | 0.5 | 87.2 | 85.7 | 83.2 |
| Example 10 | Same as Example 1 | 98 | 99.99 | 99.2 | 0.8 | 39 | 0.5 | 87.5 | 86.2 | 84.6 |
| Example 11 | Same as Example 1 | 92 | 99.98 | 99.2 | 0.8 | 71 | 0.5 | 87.0 | 85.2 | 82.6 |
| Example 12 | Same as Example 1 | 87 | 99.82 | 99.1 | 0.9 | 98 | 0.5 | 86.9 | 85.0 | 81.8 |

**[Table 4-2] Partial differences and test results of Examples 13 to 15**

| Item | Final product | | | | | | Mass percentage of final product introduced as additive in electrolyte /% | Cycling capacity retention rate at 25°C /% | Cycling capacity retention rate at 45°C /% | Storage capacity retention rate at 60°C /% |
|---|---|---|---|---|---|---|---|---|---|---|
| | Specific structural formula of product represented by Formula I | Yield /% | Purity /% | Content percentage of cis-isomer represented by Formula II in product | Content percentage of trans-isomer represented by Formula III in product /% | Total content of chlorine-containing organic compound / ppm | | | | |
| Example 13 | 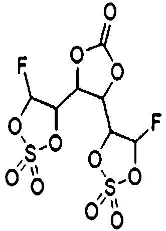 | 86 | 99.50 | 99.3 | 0.7 | 240 | 0.5 | 86.3 | 84.8 | 80.2 |
| Example 14 | 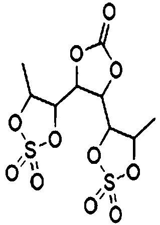 | 87 | 99.62 | 99.3 | 0.7 | 242 | 0.5 | 86.1 | 84.6 | 83.8 |
| Example 15 | 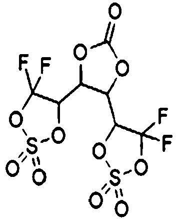 | 81 | 99.56 | 99.4 | 0.6 | 255 | 0.5 | 87.3 | 85.1 | 82.8 |

**[Table 4-3] Partial differences and test results of Examples 16 to 28**

| Item | Final product | | | | | | Mass percentage of final product introduced as additive in electrolyte /% | Cycling capacity retention rate at 25°C /% | Cycling capacity retention rate at 45°C /% | Storage capacity retention rate at 60°C /% |
|---|---|---|---|---|---|---|---|---|---|---|
| | Specific structural formula of product represented by Formula I | Yield /% | Purity /% | Content percentage of cis-isomer represented by Formula II in product | Content percentage of trans-isomer represented by Formula III in product /% | Total content of chlorine-conta ining organic compound / ppm | | | | |
| Example 16 | 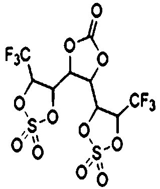 | 88 | 99.67 | 99.1 | 0.9 | 268 | 0.5 | 87.1 | 84.3 | 81.4 |
| Example 17 | 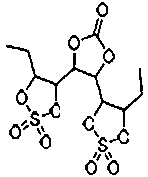 | 84 | 99.58 | 99.2 | 0.8 | 286 | 0.5 | 85.7 | 84.8 | 82.1 |
| Example 18 | Same as Example 1 | 53 | 60.00 | 99.2 | 0.8 | 2430 | 0.5 | 70.2 | 67.5 | 65.8 |
| Example 19 | Same as Example 1 | 14 | 18.93 | 99.3 | 0.7 | 3700 | *0.5* | 53.4 | 53.8 | 54.3 |
| Example 20 | Same as Example 1 | 29 | 42.00 | 99.2 | 0.8 | 3050 | 0.5 | 60.3 | 58.6 | 56.8 |
| Example 21 | Same as Example 1 | 49 | 57.50 | 99.1 | 0.9 | 2173 | 0.5 | 63.8 | 60.5 | 61.2 |
| Example 22 | Same as Example 1 | 56 | 60.98 | 99.1 | 0.9 | 560 | 0.5 | 75.8 | 74.1 | 73.9 |
| Example 23 | Same as Example 9 | 92 | 99.98 | 99.1 | 0.9 | 68 | 5 | 76.1 | 75.0 | 77.7 |
| Example 24 | Same as Example 9 | 92 | 99.98 | 99.1 | 0.9 | 68 | 3 | 80.2 | 76.5 | 80.4 |
| Example 25 | Same as Example 9 | 92 | 99.98 | 99.1 | 0.9 | 68 | 1.5 | 84.1 | 80.8 | 83.2 |
| Example 26 | Same as Example 9 | 92 | 99.98 | 99.1 | 0.9 | 68 | 0.1 | 74.3 | 73.4 | 75.2 |
| Example 27 | Same as Example 9 | 92 | 99.98 | 99.1 | 0.9 | 68 | 0.5 | 83.2 | 82.4 | 83.1 |
| Example 28 | Same as Example 9 | 92 | 99.98 | 99.1 | 0.9 | 68 | 0.5 | 82.1 | 81.5 | 82.3 |

**[Table 4-4] Partial differences and test results of Comparative Examples 1 to 10**

| Item | Final product | | | | | | Mass percentage of final product introduced as additive in electrolyte /% | Cycling capacity retention rate at 25°C /% | Cycling capacity retention rate at 45°C /% | Storage capacity retention rate at 60°C /% |
|---|---|---|---|---|---|---|---|---|---|---|
| | Specific structural formula of product represented by Formula 1 | Yield /% | Purity /% | Content percentage of cis-isomer represented by Formula II in product | Content percentage of trans-isomer represented by Formula III in product /% | Total content of chlorine-containing organic compound / ppm | | | | |
| Comparative Example 1 | Same as Example 1 | 88 | 99.92 | 95.2 | 4.8 | 69 | 0.5 | 78.5 | 76.6 | 74.8 |
| Comparative Example 2 | Same as Example 1 | 87 | 99.70 | 50.5 | 49.5 | 123 | 0.5 | 70.3 | 66.8 | 68.3 |
| Comparative Example 3 | Same as Example 1 | 86 | 99.51 | 1.1 | 98.9 | 226 | 0.5 | 63.7 | 60.3 | 61.2 |
| Comparative Example 4 | Same as Example 1 | 52 | 60.30 | 50.3 | 49.7 | 2587 | 0.5 | 67.3 | 63.2 | 61.4 |
| Comparative Example 5 | Same as Example 1 | 13 | 18.67 | 51.2 | 48.8 | 3896 | 0.5 | 51.3 | 50.9 | 52.1 |
| Comparative Example 6 | Same as Example 1 | 28 | 40.98 | 49.8 | 50.2 | 3210 | 0.5 | 57.8 | 56.2 | 54.8 |
| Comparative Example 7 | Same as Example 1 | 47 | 56.31 | 46.7 | 53.3 | 2306 | 0.5 | 61.5 | 58.7 | 59.3 |
| Comparative Example 8 | Same as Example 1 | 55 | 60.33 | 50.1 | 49.9 | 768 | 0.5 | 73.5 | 72.6 | 71.2 |
| Comparative Example 9 | Same as Example 27 | 86 | 99.51 | 1.1 | 98.9 | 226 | 0.5 | 77.8 | 72.5 | 73.1 |
| Comparative Example 10 | Same as Example 28 | 86 | 99.51 | 1.1 | 98.9 | 226 | 0.5 | 75.7 | 70.7 | 71.1 |

Note: in Table 4-1, "N.D" indicates that the substance is above the detection limit and not detected.

### Results and Conclusions:

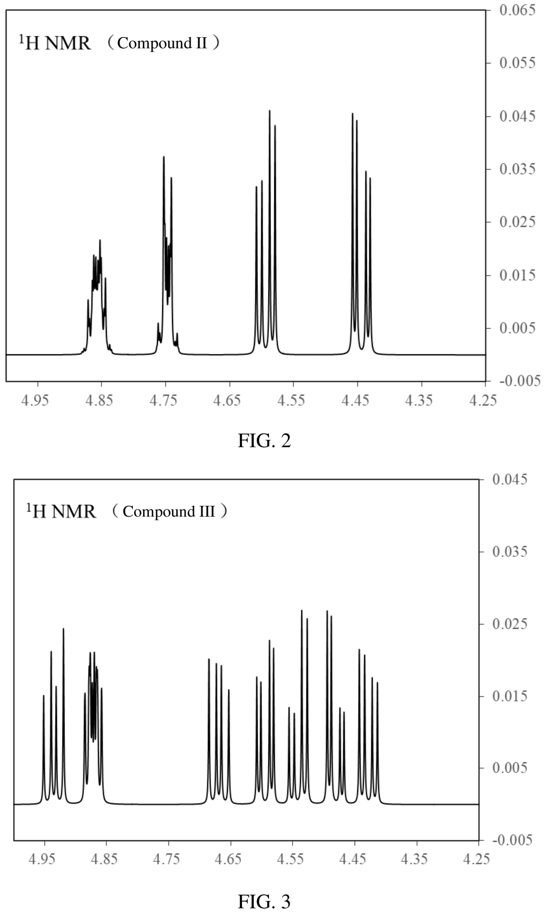

Based on the data in Table 3-1, Table 3-2, Table 3-3, Table 4-1, Table 4-2, Table 4-3, and Table 4-4, it can be seen that the target product prepared by the method of the above examples of the present disclosure is mainly dominated by the cis-isomer, while a mass percentage of the trans-isomer is relatively low, generally below 2%. Taking Example 1 as an example, FIG. 2 shows the hydrogen nuclear magnetic resonance spectrum of the final product prepared in Example 1. Compared with the reaction in Comparative Example 3, which uses the hexahydric alcohol mainly containing the trans-structure as the raw material (FIG. 3 shows the hydrogen nuclear magnetic resonance spectrum of the final product prepared in Comparative Example 3), a mass percentage of the cis-isomer in the product of Example 1 is higher, reaching 98.2%. In addition, based on Example 1 to Example 8 and Comparative Example 1 to Comparative Example 3, it can be seen that the content of the cis-isomer target product can be increased by controlling the purity of mannitol. Moreover, based on Example 9 to Example 11 and Example 18 to Example 22, it can be seen that by controlling process conditions, the selectivity of the target product can be further improved, and the content of impurities, especially the content of the chlorine-containing organic compound, can be reduced. Under the same conditions, performing the transesterification reaction under specific heating and pressurization conditions can improve the selectivity and yield of the target product, and appropriately increasing the pressure is also beneficial to further improving the selectivity and yield of the target product.

Based on Example 1 to Example 5 and Comparative Example 1 to Comparative Example 3, it can be seen that the higher the content of the cis-isomer represented by Formula II in the final product, the lower the content of the trans-isomer represented by Formula III, and the better performance of the final product in battery applications. Based on Example 6 to Example 8, it can be seen that when the content of the trans-isomer represented by Formula III in the compound represented by Formula I is lower than 0.01%, the effect of the final product on improving the battery performance becomes insignificant as the content of the trans-isomer represented by Formula III decreases. Based on Example 9 to Example 11 and Example 18 to Example 22, it can be seen that the purity of the final product also affects the performance of the battery when applied to the battery. If the purity is too low, a significant negative impact may occur on the battery performance. Based on Example 18 to Example 22 and Comparative Example 4 to Comparative Example 8, it can be seen that under the same preparation conditions, the higher the content of the trans-isomer in the raw material hexahydric alcohol, the worse the performance of the battery when the prepared final product is applied to the battery. Also, Example 1, Example 27, Example 28, Comparative Example 1 to Comparative Example 3, and Comparative Example 9 to Comparative Example 10 may prove that the product of the present disclosure has good effects when applied to different battery systems. Also, it was found by the inventor that under different battery systems, different degrees of transition metal dissolution may have different effects on the battery performance. In a battery system with severe dissolution (e.g., a battery system under a high voltage (4.35 V) and a 3C/1C fast charging condition), controlling the content of the cis-isomer represented by Formula II in the compound represented by Formula I to be greater than or equal to 98 wt% may have a more prominent effect on improving the battery performance.

In the description of the present disclosure, the description with reference to the terms "one embodiment", "some embodiments", "an example", "a specific example", or "some examples", etc., means that specific features, structures, materials, or characteristics described in conjunction with the embodiment(s) or example(s) are included in at least one embodiment or example of the present disclosure. In the present disclosure, any illustrative reference of the above terms does not necessarily refer to the same embodiment(s) or example(s). Moreover, the specific features, structures, materials, or characteristics as described can be combined in any one or more embodiments or examples as appropriate. In addition, different embodiments or examples and features of different embodiments or examples described in the specification may be combined by those skilled in the art without mutual contradiction.

Although the embodiments of the present disclosure have been shown and described above, it should be understood that the above-mentioned embodiments are exemplary and should not be construed as limiting the present disclosure. Those skilled in the art can make changes, modifications, substitutions, and alternations to the above-mentioned embodiments within the scope of the present disclosure.

## Claims

1. A polycyclic compound, comprising:
a compound represented by Formula I, wherein in the compound represented by Formula I, a content of a cis-isomer represented by Formula II is greater than or equal to 98 wt%, 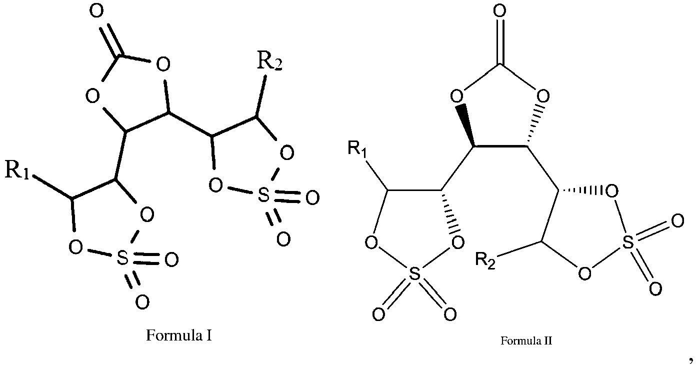
wherein R₁ and R₂ are each independently selected from any one of H, F, alkyl, or fluoroalkyl.

2. The polycyclic compound according to claim 1, satisfying at least one of the following conditions:
(i) the polycyclic compound further comprises a chlorine-containing organic compound, wherein based on a mass of the polycyclic compound, a total content of the chlorine-containing organic compound is less than or equal to 300 ppm;
(ii) the polycyclic compound further comprises other organic compounds being selected from one or more of the following 6 compounds: 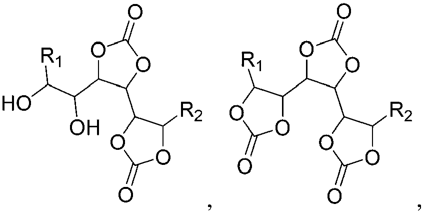 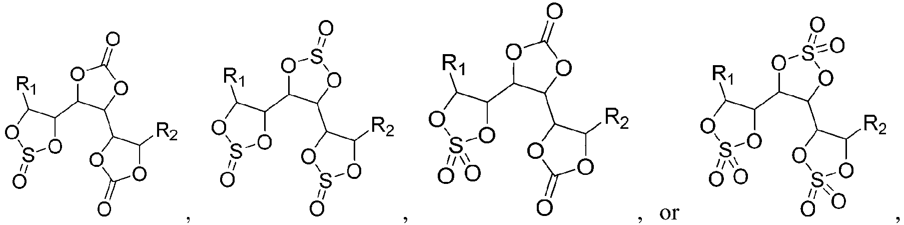 wherein based on the mass of the polycyclic compound, a total content of the other organic compounds is less than or equal to 1000 ppm;
(iii) based on the mass of the polycyclic compound, a content of the compound represented by Formula I is greater than or equal to 99 wt%;
(iv) in the compound represented by Formula I, the content of the cis-isomer represented by Formula II is greater than or equal to 98.9 wt%;
(v) the compound represented by Formula I further comprises a trans-isomer represented by Formula III, wherein in the compound represented by Formula I, a content of the trans-isomer represented by Formula III is less than or equal to 1%, 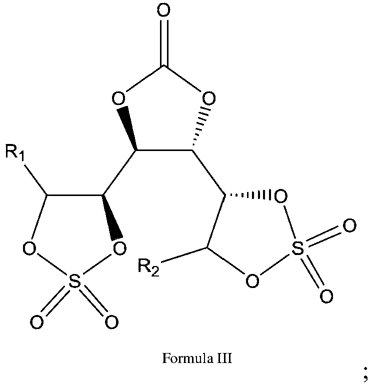 or
(vi) R₁ and R₂ are each independently selected from any one of H, F, or fluoromethyl.

3. The polycyclic compound according to claim 2, satisfying at least one of the following conditions:
(a) the chlorine-containing organic compound comprises a chlorine-containing organic compound without cyclic sulfate groups and cyclic carbonate groups, a chlorine-containing organic compound containing only cyclic carbonate groups, a chlorine-containing organic compound containing only cyclic sulfate groups, or a chlorine-containing organic compound containing 1 to 2 cyclic carbonate groups and 1 cyclic sulfate group;
(b) based on the mass of the polycyclic compound, the total content of the chlorine-containing organic compound is less than or equal to 100 ppm;
(c) based on the mass of the polycyclic compound, the total content of the other organic compounds is less than or equal to 200 ppm;
(d) based on the mass of the polycyclic compound, the content of the compound represented by Formula I is greater than or equal to 99.9 wt%; or
(e) in the compound represented by Formula I, the content of the trans-isomer represented by Formula III is less than or equal to 0.5 wt%.

4. The polycyclic compound according to claim 2 or 3, satisfying at least one of the following conditions:
(α) the chlorine-containing organic compound comprises one or more of 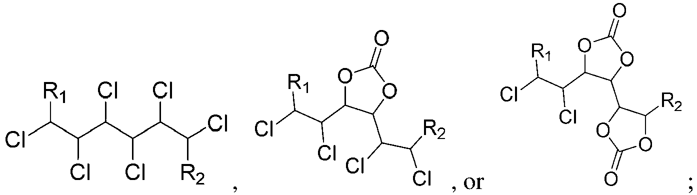
(β) based on the mass of the polycyclic compound, the total content of the chlorine-containing organic compound is less than or equal to 50 ppm;
(γ) in the compound represented by Formula I, the content of the cis-isomer represented by Formula II is greater than or equal to 99.9 wt%;
(δ) in the compound represented by Formula I, the content of the trans-isomer represented by Formula III is less than or equal to 0.1%;
(ε) the compound represented by Formula I comprises 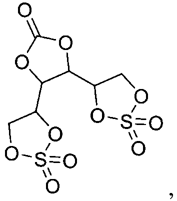 and the compound represented by Formula II comprises 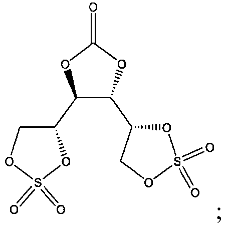
(ζ) in the compound represented by Formula I, the content of the trans-isomer represented by Formula III is greater than or equal to 0.01 wt%; or
(η) in the compound represented by Formula I, the content of the cis-isomer represented by Formula II is less than or equal to 99.99 wt%.

5. A method for preparing a polycyclic compound, the method comprising:
(1) mixing a hexahydric alcohol, a monohydric alcohol, a carbonate, and a basic catalyst, and performing a transesterification reaction, to obtain a compound 1;
(2) dissolving the compound 1 in a solvent, and performing a condensation reaction with thionyl chloride, to obtain a compound 2; and
(3) reacting the compound 2 with an oxidizing agent, to obtain a compound represented by Formula I, wherein:
structural formulas of the compound 1 and the compound 2 are represented by 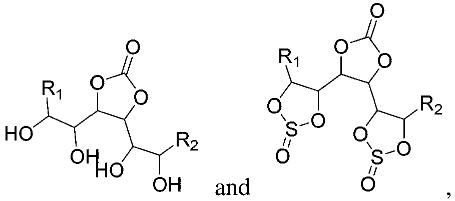 respectively, the hexahydric alcohol comprises 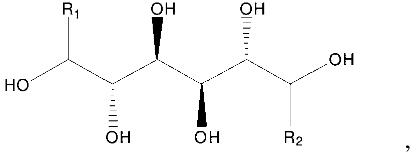 and a mass percentage of 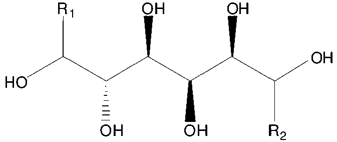 in the hexahydric alcohol is less than or equal to 2%, where R₁ and R₂ are each independently selected from any one of H, F, alkyl, or fluoroalkyl, and
the compound represented by Formula I is

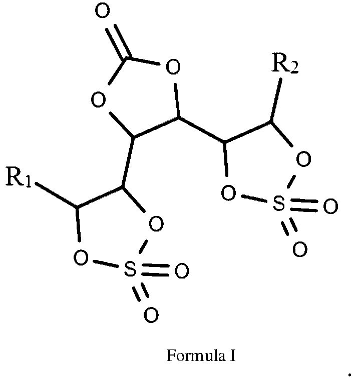

6. The method according to claim 5, wherein said mixing the hexahydric alcohol, the monohydric alcohol, the carbonate, and the basic catalyst, and performing the transesterification reaction satisfies at least one of the following conditions:
(A) subsequent to completion of the transesterification reaction, the method further comprises: performing a reduced-pressure treatment to remove a low-boiling component;
(B) the transesterification reaction is performed at a temperature ranging from 65°C to 100°C under a pressure controlled to be less than or equal to 2.5 MP for 0.5 hours to 6 hours;
(C) a molar ratio of the carbonate to the hexahydric alcohol ranges from 3:1 to 5:1;
(D) a molar ratio of the monohydric alcohol to the hexahydric alcohol ranges from 10:1 to 20:1;
(E) based on a mass of the hexahydric alcohol, an amount of the basic catalyst ranges from 0.01 wt% to 5 wt%;
(F) the carbonate comprises one or more of dimethyl carbonate, diethyl carbonate, ethyl methyl carbonate, or diphenyl carbonate;
(G) the monohydric alcohol comprises one or more of methanol, ethanol, propanol, isopropanol, butanol, or tert-butanol;
(H) the basic catalyst comprises one or more of sodium hydroxide, potassium hydroxide, potassium carbonate, sodium carbonate, lithium carbonate, sodium methoxide, sodium ethoxide, sodium tert-butoxide, potassium tert-butoxide, triethylamine, or pyridine;
(I) a mass percentage of 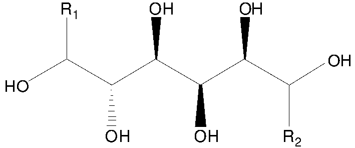 in the hexahydric alcohol is less than or equal to 1%; or
(J) the mass percentage of 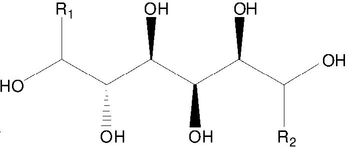 in the hexahydric alcohol is greater than or equal to 0.01%.

7. The method according to claim 5 or 6, satisfying at least one of the following conditions:
(I) in the step (2), subsequent to completion of the condensation reaction, the method further comprises: performing a reflux deacidification treatment, optionally, a temperature of the reflux deacidification treatment ranges from 40°C to 80°C; and/or a duration of the reflux deacidification treatment ranges from 10 minutes to 50 minutes;
(II) in the step (2), subsequent to dissolving the compound 1 in the solvent, the condensation reaction is performed by adding thionyl chloride dropwise;
(III) in the step (2), the condensation reaction is performed at a temperature ranging from 20°C to 100°C for 1 hour to 4 hours;
(IV) in the step (2), a molar ratio of the compound 1 to the thionyl chloride is 1:(2 to 3), and an addition duration for the thionyl chloride ranges from 0.5 hours to 2 hours;
(V) in the step (2), the solvent comprises one or more of an ether solvent, N,N-dimethylformamide, N,N-dimethylacetamide, tetrahydrofuran, or an ester solvent; or
(VI) in the step (3), the oxidizing agent is provided as an aqueous solution, and subsequent to reacting the compound 2 with the oxidizing agent, the method further comprises: performing a heating treatment on a reaction product to remove water.

8. The method according to any one of claims 5 to 7, wherein said mixing the hexahydric alcohol, the monohydric alcohol, the carbonate, and the basic catalyst, and performing the transesterification reaction satisfies at least one of the following conditions:
1) the transesterification reaction is performed at a temperature ranging from 70°C to 90°C and a pressure ranging from 0.5 MPa to 2.2 MPa;
2) a molar ratio of the monohydric alcohol to the hexahydric alcohol ranges from 12:1 to 18:1;
3) based on a mass of the hexahydric alcohol, an amount of the basic catalyst ranges from 0.02 wt% to 0.5 wt%; or
4) the mass percentage of 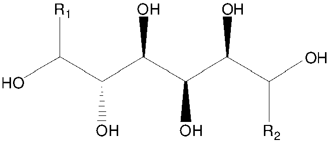 in the hexahydric alcohol is less than or equal to 0.5%.

9. The method according to any one of claims 5 to 8, wherein said mixing the hexahydric alcohol, the monohydric alcohol, the carbonate, and the basic catalyst, and performing the transesterification reaction satisfies at least one of the following conditions:
(1) the transesterification reaction is performed at a temperature ranging from 75°C to 85°C and a pressure ranging from 1 MPa to 2 MPa;
(2) a molar ratio of the monohydric alcohol to the hexahydric alcohol ranges from 14:1 to 16:1; and
(3) based on a mass of the hexahydric alcohol, an amount of the basic catalyst ranges from 0.02 wt% to 0.1 wt%; or
(4) the mass percentage of 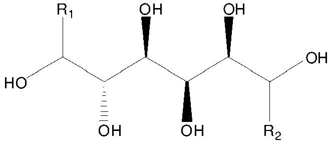 in the hexahydric alcohol is less than or equal to 0.1%.

10. A polycyclic compound prepared by the method according to any one of claims 5 to 9, the polycyclic compound comprising:
the compound represented by Formula I, wherein in the compound represented by Formula I, a content of a cis-isomer represented by Formula II is greater than or equal to 98 wt%, and the cis-isomer represented by Formula II is

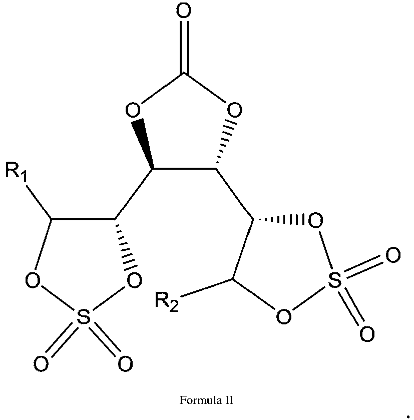

11. Use of the polycyclic compound according to any one of claims 1 to 4 or the polycyclic compound prepared by the method according to any one of claims 5 to 9 in the field of electrolyte and battery.

12. An electrolyte, comprising an electrolyte additive, wherein the electrolyte additive comprises:
the polycyclic compound according to any one of claims 1 to 4; or
the polycyclic compound prepared by the method according to any one of claims 5 to 9.

13. The electrolyte according to claim 12, wherein based on a total mass of the electrolyte, a content of the electrolyte additive ranges from 0.1 wt% to 5 wt%.

14. A battery, comprising the electrolyte according to claim 12 or 13.
